# EUROPÄISCHE PATENTANMELDUNG

(11) **EP 4 241 672 A2**
(43) Veröffentlichungstag der Anmeldung: **13.09.2023**
(21) Anmeldenummer: 23178079.2
(22) Anmeldetag: 05.10.2020
(51) Int. Cl.: A61B 5/055

(54) **BESCHLEUNIGUNG VON MRT-UNTERSUCHUNGEN**

(30) Priorität: 11.10.2019 EP 19202711; 06.05.2020 EP 20173089
(62) Teilanmeldung aus: 20785507.3
(71) Anmelder: Bayer Aktiengesellschaft, 51373 Leverkusen (DE)
(72) Erfinder: Knobloch, Gesine, 13507 Berlin (DE); Lienerth, Christian, 60486 Frankfurt am Main (DE)
(74) Vertreter: BIP Patents

(57) **Zusammenfassung**

Die vorliegende Erfindung befasst sich mit der Beschleunigung von MRT-Untersuchungen, insbesondere bei der Detektion und Differentialdiagnose fokaler Leberläsionen mittels dynamischer kontrastverstärkender Magnetresonanztomographie (MRT). Gegenstände der vorliegenden Erfindung sind ein Verfahren, ein System und ein Computerprogrammprodukt zur Erzeugung von MRT-Bildern, insbesondere der Leber.

## Beschreibung

Die vorliegende Erfindung befasst sich mit der Beschleunigung von MRT-Untersuchungen, insbesondere bei der Detektion und Differentialdiagnose fokaler Leberläsionen mittels dynamischer kontrastverstärkender Magnetresonanztomographie (MRT). Gegenstände der vorliegenden Erfindung sind insbesondere ein Verfahren, ein System und ein Computerprogrammprodukt zur Erzeugung von MRT-Bildern, insbesondere der Leber.

Die Magnetresonanztomographie, abgekürzt MRT oder MR (engl. MRI: *Magnetic Resonance Imaging*), ist ein bildgebendes Verfahren, das vor allem in der medizinischen Diagnostik zur Darstellung von Struktur und Funktion der Gewebe und Organe im menschlichen oder tierischen Körper eingesetzt wird.

Bei der MR-Bildgebung werden die magnetischen Momente von Protonen in einem Untersuchungsobjekt in einem Grundmagnetfeld ausgerichtet, so dass sich eine makroskopische Magnetisierung entlang einer Längsrichtung einstellt. Diese wird anschließend durch das Einstrahlen von Hochfrequenz(HF)-Pulsen aus der Ruhelage ausgelenkt (Anregung). Die Rückkehr der angeregten Zustände in die Ruhelage (Relaxation) bzw. die Magnetisierungsdynamik wird anschließend mittels einer oder mehrerer HF-Empfangsspulen als Relaxationssignale detektiert.

Zur Ortskodierung werden dem Grundmagnetfeld schnell geschaltete magnetische Gradientenfelder überlagert. Die erfassten Relaxationssignale bzw. die detektierten und ortsaufgelösten MR-Daten liegen zunächst als Rohdaten in einem Ortsfrequenzraum vor, und können durch anschließende FourierTransformation in den Ortsraum (Bildraum) transformiert werden.

Bei der nativen MRT werden die Gewebs-Kontraste durch die unterschiedlichen Relaxationszeiten (T1 und T2) und die Protonendichte erzeugt.

Die T1-Relaxation beschreibt den Übergang der Längsmagnetisierung (longitudinale Magnetisierung) in ihren Gleichgewichtszustand, wobei T1 diejenige Zeit ist, die benötigt wird, um 63,21% der Gleichgewichtsmagnetisierung vor der Resonanzanregung zu erreichen. Sie wird auch longitudinale Relaxaktionszeit oder Spin-Gitter-Relaxationszeit genannt.

Die T2-Relaxation beschreibt in analoger Weise den Übergang der Transversalmagnetisierung in ihren Gleichgewichtszustand.

MR-Kontrastmittel entfalten ihre Wirkung, indem sie die Relaxationszeiten der Strukturen, die Kontrastmittel aufnehmen, verändern. Es lassen sich zwei Stoffgruppen unterscheiden: para- und superparamagnetische Stoffe. Beide Stoffgruppen besitzen ungepaarte Elektronen, die ein magnetisches Feld um die einzelnen Atome bzw. Moleküle induzieren.

Superparamagnetische führen zu einer überwiegenden T2-Verkürzung, während paramagnetische Kontrastmittel im Wesentlichen zu einer T1-Verkürzung führen. Eine Verkürzung der T1-Zeit führt zu einer Zunahme der Signalintensität in T1-gewichteten MRT-Aufnahmen, eine Verkürzung der T2-Zeit führt zu einer Abnahme der Signalintensität in T2-gewichteten MRT-Aufnahmen.

Die Wirkung dieser Kontrastmittel ist indirekt, da das Kontrastmittel selbst kein Signal abgibt, sondern nur die Signalintensität der Wasserstoffprotonen in seiner Umgebung beeinflusst.

Ein Beispiel für ein superparamagnetisches Kontrastmittel sind Eisenoxidnanopartikel (SPIO, engl.: *superparamagnetic iron oxide*)*.*

Beispiele für paramagnetische Kontrastmittel sind Gadolinium-Chelate wie Gadopentetat-Dimeglumin (Handelsname: Magnevist^{®} u.a.), Gadobenat-Dimeglumin (Handelsname: Multihance^{®}), Gadotersäure (Dotarem^{®}, Dotagita^{®}, Cyclolux^{®}), Gadodiamid (Omniscan^{®}), Gadoteridol (ProHance^{®}) und Gadobutrol (Gadovist^{®}).

Nach ihrem Verteilungsmuster im Gewebe können extrazelluläre, intrazelluläre und intravaskuläre Kontrastmittel unterschieden werden.

Kontrastmittel auf der Basis der Gadoxetsäure zeichnen sich dadurch aus, dass sie spezifisch von Leberzellen, den Hepatozyten, aufgenommen werden, sich im Funktionsgewebe (Parenchym) anreichern und die Kontraste in gesundem Lebergewebe verstärken. Die Zellen von Zysten, Metastasen und der meisten Leberzellkarzinome arbeiten nicht mehr wie normale Leberzellen, nehmen das Kontrastmittel nicht oder kaum auf, werden nicht verstärkt dargestellt und werden damit erkennbar und lokalisierbar.

Beispiele für Kontrastmittel auf der Basis der Gadoxetsäure sind in US 6,039,931A beschrieben; sie sind kommerziell zum Beispiel unter den Markennamen Primovist^{®} oder Eovist^{®} erhältlich.

Der kontrastverstärkende Effekt von Primovist^{®}/Eovist^{®} wird durch den stabilen Gadoliniumkomplex Gd-EOB-DTPA (Gadolinium-Ethoxybenzyl-Diethylentriamin-Pentaessigsäure) vermittelt. DTPA bildet mit dem paramagnetischen Gadoliniumion einen Komplex, der eine extrem hohe thermodynamische Stabilität aufweist. Der Ethoxybenzylrest (EOB) ist der Vermittler der hepatobiliären Aufnahme des Kontrastmittels.

Primovist^{®} kann zur Detektion von Tumoren in der Leber eingesetzt werden. Die Blutversorgung des gesunden Lebergewebes erfolgt in erster Linie über die Pfortader (*Vena portae*), während die Leberarterie (*Arteria hepatica*) die meisten Primärtumoren versorgt. Nach intravenöser Bolusinjektion eines Kontrastmittels lässt sich dementsprechend eine Zeitverzögerung zwischen der Signalanhebung des gesunden Leberparenchyms und des Tumors beobachten.

Neben malignen Tumoren findet man in der Leber häufig gutartige Läsionen wie Zysten, Hämangiome und fokal noduläre Hyperplasien (FNH). Für eine sachgerechte Therapieplanung müssen diese von den malignen Tumoren differenziert werden. Primovist^{®} kann zur Erkennung gutartiger und bösartiger fokaler Leberläsionen eingesetzt werden. Es liefert mittels T1-gewichteter MRT Informationen über den Charakter dieser Läsionen. Bei der Differenzierung nutzt man die unterschiedliche Blutversorgung von Leber und Tumor und den zeitlichen Verlauf der Kontrastverstärkung.

Die mittels Primovist^{®} erzielte Kontrastverstärkung kann man in mindestens zwei Phasen unterteilen: in eine dynamische Phase (umfassend die so genannte arterielle Phase, portalvenöse Phase und Spätphase) und die hepatobiliäre Phase, in der bereits eine signifikante Aufnahme von Primovist^{®} in die Hepatozyten stattgefunden hat.

Die zeitliche Verfolgung der Verteilung des Kontrastmittels über die dynamische Phase und die hepatobiliäre Phase bietet auf der einen Seite eine gute Möglichkeit der Detektion und Differentialdiagnose fokaler Leberläsionen; auf der anderen Seite zieht sich die Untersuchung jedoch über eine vergleichsweise lange Zeitspanne hin. Über diese Zeitspanne sollten Bewegungen des Patienten vermieden werden, um Bewegungsartefakte in den MRT-Aufnahme zu minimieren. Die lang andauernde Bewegungseinschränkung kann für einen Patienten unangenehm sein.

Ausgehend vom beschriebenen Stand der Technik bestand die technische Aufgabe darin, die Zeitspanne der MRT-Untersuchung zu verringern.

Diese Aufgabe wird durch die Gegenstände der unabhängigen Patentansprüche gelöst. Bevorzugte Ausführungsformen der vorliegenden Erfindung finden sich in den abhängigen Patentansprüchen, in dieser Beschreibung und in den Zeichnungen.

Ein erster Gegenstand der vorliegenden Erfindung ist ein Verfahren umfassend die Schritte
- Empfangen einer ersten MRT-Aufnahmε eines Untersuchungsobjekts, wobei die erste MRT-Aufnahme eine Leber oder einen Teil einer Leber des Untersuchungsobjekts zu einem ersten Zeitpunkt nach einer Applikation eines ersten Kontrastmittels zeigt, wobei gesunde Leberzellen zu dem ersten Zeitpunkt infolge der Applikation des ersten Kontrastmittels in der ersten MRT-Aufnahme kontrastverstärkt dargestellt sind,
- Empfangen einer zweiten MRT-Aufnahme desselben Untersuchungsobjekts, wobei die zweite MRT-Aufnahme dieselbe Leber oder denselben Teil der Leber zu einem zweiten Zeitpunkt nach einer Applikation eines zweiten Kontrastmittels zeigt, wobei gesunde Leberzellen zu dem zweiten Zeitpunkt infolge der Applikation des ersten Kontrastmittels in der zweiten MRT-Aufnahme kontrastverstärkt dargestellt sind, und wobei Blutgefäße zu dem zweiten Zeitpunkt infolge der Applikation des zweiten Kontrastmittels in der zweiten MRT-Aufnahme kontrastverstärkt dargestellt sind,
- Erzeugen eines ersten MRT-Bildes aus der ersten MRT-Aufnahme und der zweiten MRT-Aufnahme, wobei das erste MRT-Bild dieselbe Leber oder denselben Teil der Leber zu dem zweiten Zeitpunkt zeigt, wobei der Kontrast zwischen gesunden Leberzellen und Blutgefäßen in dem ersten MRT-Bild gegenüber der zweiten MRT-Aufnahme vergrößert ist,
- Anzeigen und/oder Ausgeben des ersten MRT-Bildes und/oder Speichern des ersten MRT-Bildes in einem Datenspeicher.

Ein weiterer Gegenstand der vorliegenden Erfindung ist ein System umfassend
- eine Empfangseinheit,
- eine Steuer- und Recheneinheit und
- eine Ausgabeeinheit,
   - wobei die Steuer- und Recheneinheit konfiguriert ist, die Empfangseinheit zu veranlassen, eine erste MRT-Aufnahme eines Untersuchungsobjekts zu empfangen, wobei die erste MRT-Aufnahme eine Leber oder einen Teil einer Leber des Untersuchungsobjekts zu einem ersten Zeitpunkt nach einer Applikation eines ersten Kontrastmittels zeigt, wobei gesunde Leberzellen zu dem ersten Zeitpunkt infolge der Applikation des ersten Kontrastmittels in der ersten MRT-Aufnahme kontrastverstärkt dargestellt sind,
   - wobei die Steuer- und Recheneinheit konfiguriert ist, die Empfangseinheit zu veranlassen, eine zweite MRT-Aufnahme eines Untersuchungsobjekts zu empfangen, wobei die zweite MRT-Aufnahme dieselbe Leber oder denselben Teil der Leber zu einem zweiten Zeitpunkt nach einer Applikation eines zweiten Kontrastmittels zeigt, wobei gesunde Leberzellen zu dem zweiten Zeitpunkt infolge der Applikation des ersten Kontrastmittels in der zweiten MRT-Aufnahme kontrastverstärkt dargestellt sind, und wobei Blutgefäße zu dem zweiten Zeitpunkt infolge der Applikation des zweiten Kontrastmittels in der zweiten MRT-Aufnahme kontrastverstärkt dargestellt sind,
   - wobei die Steuer- und Recheneinheit konfiguriert ist, aus der ersten MRT-Aufnahme und der zweiten MRT-Aufnahme ein erstes MRT-Bild zu erzeugen, wobei das erste MRT-Bild dieselbe Leber oder denselben Teil der Leber zu dem zweiten Zeitpunkt zeigt, wobei der Kontrast zwischen gesunden Leberzellen und Blutgefäßen in dem ersten MRT-Bild gegenüber der zweiten MRT-Aufnahme vergrößert ist,
   - wobei die Steuer- und Recheneinheit konfiguriert ist, die Ausgabeeinheit zu veranlassen, das erste MRT-Bild anzuzeigen, auszugeben oder in einem Datenspeicher zu speichern.

Ein weiterer Gegenstand der vorliegenden Erfindung ist ein Computerprogrammprodukt umfassend ein Computerprogramm, das in einen Arbeitsspeicher eines Computersystems geladen werden kann und dort das Computersystem dazu veranlasst, folgende Schritte ausführen:
- Empfangen einer ersten MRT-Aufnahme eines Untersuchungsobjekts, wobei die erste MRT-Aufnahme eine Leber oder einen Teil einer Leber des Untersuchungsobjekts zu einem ersten Zeitpunkt nach einer Applikation eines ersten Kontrastmittels zeigt, wobei gesunde Leberzellen zu dem ersten Zeitpunkt infolge der Applikation des ersten Kontrastmittels in der ersten MRT-Aufnahme kontrastverstärkt dargestellt sind,
- Empfangen einer zweiten MRT-Aufnahme desselben Untersuchungsobjekts, wobei die zweite MRT-Aufnahme dieselbe Leber oder denselben Teil der Leber zu einem zweiten Zeitpunkt nach einer Applikation eines zweiten Kontrastmittels zeigt, wobei gesunde Leberzellen zu dem zweiten Zeitpunkt infolge der Applikation des ersten Kontrastmittels in der zweiten MRT-Aufnahme kontrastverstärkt dargestellt sind, und wobei Blutgefäße zu dem zweiten Zeitpunkt infolge der Applikation des zweiten Kontrastmittels in der zweiten MRT-Aufnahme kontrastverstärkt dargestellt sind,
- Erzeugen eines ersten MRT-Bildes aus der ersten MRT-Aufnahme und der zweiten MRT-Aufnahme, wobei das erste MRT-Bild dieselbe Leber oder denselben Teil der Leber zu dem zweiten Zeitpunkt zeigt, wobei der Kontrast zwischen gesunden Leberzellen und Blutgefäßen in dem ersten MRT-Bild gegenüber der zweiten MRT-Aufnahme vergrößert ist,
- Anzeigen und/oder Ausgeben des ersten MRT-Bildes und/oder Speichern des ersten MRT-Bildes in einem Datenspeicher.

Ein weiterer Gegenstand der vorliegenden Erfindung ist die Verwendung eines ersten und eines zweiten Kontrastmittels in einem MRT-Verfahren, wobei das MRT-Verfahren die folgenden Schritte umfasst:
- Applizieren des erstes Kontrastmittels,
- Erzeugen einer ersten MRT-Aufnahme, wobei die erste MRT-Aufnahme eine Leber oder einen Teil einer Leber zu einem ersten Zeitpunkt nach der Applikation des ersten Kontrastmittels zeigt, wobei gesunde Leberzellen zu dem ersten Zeitpunkt infolge der Applikation des ersten Kontrastmittels in der ersten MRT-Aufnahme kontrastverstärkt dargestellt sind,
- Applizieren des zweiten Kontrastmittels zu einem Zeitpunkt nach dem Erzeugen der ersten MRT-Aufnahme,
- Erzeugen einer zweiten MRT-Aufnahme, wobei die zweite MRT-Aufnahme dieselbe Leber oder denselben Teil der Leber zu einem zweiten Zeitpunkt nach der Applikation des zweiten Kontrastmittels zeigt, wobei gesunde Leberzellen zu dem zweiten Zeitpunkt infolge der Applikation des ersten Kontrastmittels in der zweiten MRT-Aufnahme kontrastverstärkt dargestellt sind, und wobei Blutgefäße zu dem zweiten Zeitpunkt infolge der Applikation des zweiten Kontrastmittels in der zweiten MRT-Aufnahme kontrastverstärkt dargestellt sind,
- Erzeugen eines ersten MRT-Bildes aus der ersten MRT-Aufnahme und der zweiten MRT-Aufnahme, wobei das erste MRT-Bild dieselbe Leber oder denselben Teil der Leber zu dem zweiten Zeitpunkt zeigt, wobei der Kontrast zwischen gesunden Leberzellen und Blutgefäßen in dem ersten MRT-Bild gegenüber der zweiten MRT-Aufnahme vergrößert ist,
- Anzeigen und/oder Ausgeben des ersten MRT-Bildes und/oder Speichern des ersten MRT-Bildes in einem Datenspeicher.

Ein weiterer Gegenstand ist ein erstes und ein zweites Kontrastmittel zur Verwendung in einem MRT-Verfahren, wobei das MRT-Verfahren die folgenden Schritte umfasst:
- Applizieren des erstes Kontrastmittels,
- Erzeugen einer ersten MRT-Aufnahme, wobei die erste MRT-Aufnahme eine Leber oder einen Teil einer Leber zu einem ersten Zeitpunkt nach der Applikation des ersten Kontrastmittels zeigt, wobei gesunde Leberzellen zu dem ersten Zeitpunkt infolge der Applikation des ersten Kontrastmittels in der ersten MRT-Aufnahme kontrastverstärkt dargestellt sind,
- Applizieren des zweiten Kontrastmittels zu einem Zeitpunkt nach dem Erzeugen der ersten MRT-Aufhahme,
- Erzeugen einer zweiten MRT-Aufnahme, wobei die zweite MRT-Aufhahme dieselbe Leber oder denselben Teil der Leber zu einem zweiten Zeitpunkt nach der Applikation des zweiten Kontrastmittels zeigt, wobei gesunde Leberzellen zu dem zweiten Zeitpunkt infolge der Applikation des ersten Kontrastmittels in der zweiten MRT-Aufhahme kontrastverstärkt dargestellt sind, und wobei Blutgefäße zu dem zweiten Zeitpunkt infolge der Applikation des zweiten Kontrastmittels in der zweiten MRT-Aufhahme kontrastverstärkt dargestellt sind,
- Erzeugen eines ersten MRT-Bildes aus der ersten MRT-Aufnahme und der zweiten MRT-Aufnahme, wobei das erste MRT-Bild dieselbe Leber oder denselben Teil der Leber zu dem zweiten Zeitpunkt zeigt, wobei der Kontrast zwischen gesunden Leberzellen und Blutgefäßen in dem ersten MRT-Bild gegenüber der zweiten MRT-Aufnahme vergrößert ist,
- Anzeigen und/oder Ausgeben des ersten MRT-Bildes und/oder Speichern des ersten MRT-Bildes in einem Datenspeicher.

Ein weiterer Gegenstand ist ein Kit umfassend ein Kontrastmittel und ein erfindungsgemäßes Computerprogrammprodukt.

Die Erfindung wird nachstehend näher erläutert, ohne zwischen den Erfindungsgegenständen (Verfahren, System, Computerprogrammprodukt, Verwendung, Kontrastmittel zur Verwendung, Kit) zu unterscheiden. Die nachfolgenden Erläuterungen sollen vielmehr für alle Erfindungsgegenstände in analoger Weise gelten, unabhängig davon, in welchem Kontext (Verfahren, System, Computerprogrammprodukt, Verwendung, Kontrastmittel zur Verwendung, Kit) sie erfolgen.

Bei einer klassischen MRT-Untersuchung der Leber oder eines Teils der Leber eines Untersuchungsobjekts wird ein hepatobiliäres Kontrastmittel in Form eines einzigen Bolus appliziert. Anschließend werden eine Reihe von MRT-Aufnahmen erzeugt, die die Leber oder den Teil der Leber während der arteriellen Phase, der portalvenösen Phase, der Spätphase und der hepatobiliären Phase zeigen. Üblicherweise befindet sich das Untersuchungsobjekt bei der Applikation des Kontrastmittels bereits in dem Kernspintomographen. Da sich das Kontrastmittel nach der intravenösen Verabreichung als Bolus vergleichsweise langsam in dem Untersuchungsbereich verteilt, zieht sich die MRT-Untersuchung über einen vergleichsweise langen Zeitraum hin. Üblich sind etwa zehn bis zwanzig Minuten.

Erfindungsgemäß wird dieser Untersuchungszeitraum verkürzt, indem Kontrastmittel in Form von zwei Boli appliziert wird, wobei sich das Untersuchungsobjekt zum Zeitpunkt der ersten Applikation noch nicht im Kernspintomographen befindet.

Es wird ein erstes Kontrastmittel als Bolus appliziert, während sich das Untersuchungsobjekt noch nicht im Kernspintomographen befindet und es wird ein zweites Kontrastmittel als Bolus appliziert, wenn sich das Untersuchungsobjekt im Kernspintomographen befindet. Das erste Kontrastmittel ist ein hepatobiliäres Kontrastmittel; das zweite Kontrastmittel kann das gleiche Kontrastmittel wie das erste Kontrastmittel sein; es kann sich bei dem zweiten Kontrastmittel aber auch um ein anderes, insbesondere um ein extrazelluläres Kontrastmittel handeln.

Die MRT-Untersuchung beginnt erst zu einem Zeitpunkt in einer (ersten) hepatobiliären Phase, bei dem das erste Kontrastmittel bereits zu einer Kontrastverstärkung von gesundem Lebergewebe führt. Zu diesem Zeitpunkt, der in dieser Beschreibung auch als erster Zeitpunkt bezeichnet wird, wird eine erste MRT-Aufnahme erzeugt. Erst zu diesem Zeitpunkt muss sich das Untersuchungsobjekt in dem Kernspintomographen befinden. Zu diesem ersten Zeitpunkt sind die arterielle Phase, die portalvenöse Phase und die Spätphase infolge der Applikation des ersten Kontrastmittels bereits vergangen; daher konnten keine MRT-Aufnahmen erzeugt werden, die die Leber oder den Teil der Leber während dieser arteriellen Phase, dieser portalvenösen Phase und/oder dieser Spätphase zeigen. Um trotzdem MRT-Aufnahmen von diesen Phasen zu erhalten, wird ein zweites Kontrastmittel verabreicht und es wird die Verbreitung des zweiten Kontrastmittels über die Blutgefäße mittels MRT verfolgt. Bei den MRT-Aufnahmen, die nach der Applikation des zweiten Kontrastmittels erzeugt werden, befindet sich noch erstes Kontrastmittel in den gesunden Leberzellen, so dass der Kontrast zwischen den Blutgefäßen und den gesunden Leberzellen gering ist. Um den Kontrast zwischen den gesunden Leberzellen und den Blutgefäßen zu erhöhen, werden aus den MRT-Aufnahmen nach der Applikation des zweiten Kontrastmittels unter Verwendung eines oder mehrerer MRT-Aufnahmen vor der Applikation des zweiten Kontrastmittels aber nach der Applikation des ersten Kontrastmittels künstliche MRT-Bilder mit einem erhöhten Kontrast erzeugt. In einer Ausführungsform erfolgt dies durch Subtraktion einer MRT-Aufnahme, die während der hepatobiliären Phase nach der Applikation des ersten Kontrastmittels und vor der Applikation des zweiten Kontrastmittels erzeugt worden ist, von einer oder mehreren MRT-Aufnahmen, die während der dynamischen Phase (d.h. während der arteriellen, der portalvenösen und/oder der Spätphase) nach der Applikation des zweiten Kontrastmittels erzeugt worden ist/sind. In einer weiteren Ausführungsform erfolgt die Erzeugung künstlicher MRT-Bilder mit Hilfe eines künstlichen neuronalen Netzes. In beiden Ausführungsformen können künstliche MRT-Bilder erzeugt werden, die den Eindruck erwecken, als ob kein erstes Kontrastmittel verabreicht worden wäre, sondern nur das zweite Kontrastmittel appliziert worden wäre.

Das "Untersuchungsobjekt" ist üblicherweise ein Lebewesen, vorzugsweise ein Säugetier, ganz besonders bevorzugt ein Mensch.

Ein Teil des Untersuchungsobjekts wird einer kontrastverstärkten Magnetresonanztomographie-Untersuchung unterzogen. Der "Untersuchungsbereich", auch Aufnahmevolumen (engl.: *field of view,* FOV) genannt, stellt insbesondere ein Volumen dar, welches in den Magnetresonanzaufnahmen abgebildet wird. Der Untersuchungsbereich wird typischerweise durch einen Radiologen, beispielsweise auf einer Übersichtsaufnahme (engl.: *localizer*) festgelegt. Selbstverständlich kann der Untersuchungsbereich alternativ oder zusätzlich auch automatisch, beispielsweise auf Grundlage eines ausgewählten Protokolls, festgelegt werden. Der Untersuchungsbereich umfasst zumindest einen Teil der Leber des Untersuchungsobjekts.

Dem Untersuchungsobjekt wird ein erstes Kontrastmittel verabreicht, das sich in dem Untersuchungsbereich verteilt. Das erste Kontrastmittel wird vorzugsweise intravenös als Bolus, gewichtsadaptiert verabreicht (z.B. in eine Armvene).

Unter einem "Kontrastmittel" wird ein Stoff oder Stoffgemisch verstanden, dessen Anwesenheit in einer Magnetresonanzmessung zu einem veränderten Signal führt. Vorzugsweise führt das erste Kontrastmittel zu einer Verkürzung der T1-Relaxationszeit und/oder der T2-Relaxationszeit.

Das erste Kontrastmittel ist ein hepatobiliäres Kontrastmittel wie beispielsweise Gd-EOB-DTPA oder Gd-BOPTA.

In einer besonders bevorzugten Ausführungsform handelt es sich bei dem ersten Kontrastmittel um einen Stoff oder ein Stoffgemisch mit der Gadoxetsäure oder einem Salz der Gadoxetsäure als kontrastverstärkenden Wirkstoff. Ganz besonders bevorzugt handelt es sich um das Dinatriumsalz der Gadoxetsäure (Gd-EOB-DTPA Dinatrium).

Nach der intravenösen Applikation des hepatobiliären Kontrastmittels in Form eines Bolus in eine Armvene erreicht das Kontrastmittel die Leber zunächst über die Arterien. Diese sind in den entsprechenden MRT-Aufnahmen kontrastverstärkt dargestellt. Die Phase, in der die Leberarterien in MRT-Aufnahmen kontrastverstärkt dargestellt sind, wird als "arterielle Phase" bezeichnet. Diese Phase beginnt unmittelbar nach der Applikation des Kontrastmittels und dauert üblicherweise 15 bis 60 Sekunden.

Anschließend erreicht das Kontrastmittel die Leber über die Lebervenen. Während der Kontrast in den Leberarterien bereits abnimmt, erreicht der Kontrast in den Lebervenen ein Maximum. Die Phase, in der die Lebervenen in MRT-Aufnahmen kontrastverstärkt dargestellt sind, wird als "portalvenöse Phase" bezeichnet. Diese Phase kann bereits während der arteriellen Phase beginnen und sich mit dieser überlagern. Üblicherweise startet diese Phase 60 bis 70 Sekunden nach der intravenösen Applikation und dauert üblicherweise 50 bis 70 Sekunden.

An die portalvenöse Phase schließt sich die "Spätphase" an, in der der Kontrast in den Leberarterien weiter absinkt, der Kontrast in den Lebervenen ebenfalls absinkt. Bei Verwendung eines hepatobiliären Kontrastmittels steigt der Kontrast in den gesunden Leberzellen in der Spätphase allmählich an. Diese Phase beginnt üblicherweise 100 bis 140 Sekunden nach der Applikation des Kontrastmittels und dauert üblicherweise 50 bis 70 Sekunden.

Die arterielle Phase, die portalvenöse Phase und die Spätphase werden zusammenfassend auch als "dynamische Phase" bezeichnet.

10-20 Minuten nach seiner Injektion führt ein hepatobiliäres Kontrastmittel zu einer deutlichen Signalverstärkung im gesunden Leberparenchym. Diese Phase wird als "hepatobiliäre Phase" bezeichnet. Das Kontrastmittel wird aus den Leberzellen nur langsam ausgeschieden; dementsprechend kann die hepatobiliäre Phase zwei Stunden und mehr andauern.

Die genannten Phasen sind beispielsweise in den folgenden Publikationen näher beschrieben: J. Magn. Reson. Imaging, 2012, 35(3): 492-511, doi:10.1002/jmri.22833; Clujul Medical, 2015, Vol. 88 no. 4: 438-448, DOI: 10.15386/cjmed-414; Journal of Hepatology, 2019, Vol. 71: 534-542, http://dx.doi.org/10.1016/jjhep.2019.05.005).

Ein extrazelluläres Kontrastmittel wird nicht durch die Leberzellen aufgenommen und reichert sich nicht im gesunden Lebergewebe an. Ein extrazelluläres Kontrastmittel zeigt daher nur eine dynamische Phase (umfassend eine arterielle Phase, eine portalvenöse Phase und eine Spätphase).

Erfindungsgemäß wird Kontrastmittel in Form von zwei Boli verabreicht. Die erste Applikation erfolgt zu einem Zeitpunkt, bei dem sich das Untersuchungsobjekt noch nicht im Kernspintomographen befindet. Bei der ersten Applikation wird das erste Kontrastmittel verabreicht.

Nach der Applikation des ersten Kontrastmittels kann eine Zeitspanne gewartet werden, bevor das Untersuchungsobjekt in den Kernspintomographen eingebracht und zu einem ersten Zeitpunkt eine erste MRT-Aufnahme erzeugt wird. Die Zeitspanne zwischen der ersten Applikation und der Erzeugung der ersten MRT-Aufnahme liegt vorzugsweise im Bereich von 5 Minuten bis 1 Stunde, noch mehr bevorzugt im Bereich von 10 Minuten bis 30 Minuten am meisten bevorzugt im Bereich von 8 Minuten bis 25 Minuten.

Die erste MRT-Aufnahme zeigt die Leber oder einen Teil der Leber des Untersuchungsobjekts während der hepatobiliären Phase nach der Applikation des ersten Kontrastmittels. Dabei sind gesunde Leberzellen infolge der Applikation des ersten Kontrastmittels in der ersten MRT-Aufnahme kontrastverstärkt dargestellt.

Die hepatobiliäre Phase, in der die erste MRT-Aufnahme erzeugt wird, wird in dieser Beschreibung auch als erste hepatobiliäre Phase bezeichnet. Das erste Kontrastmittel hat die gesunden Leberzellen erreicht und führt zu einer Kontrastverstärkung, bei einem paramagnetischen Kontrastmittel zu einer Signalverstärkung der gesunden Leberzellen. Während der arteriellen Phase, der portalvenösen Phase und der Spätphase, die nach der Applikation des ersten Kontrastmittels eintreten, werden keine MRT-Aufnahmen erzeugt. Die arterielle Phase, die portalvenöse Phase und die Spätphase, die nach der Applikation des ersten Kontrastmittels eintreten, werden in dieser Beschreibung auch als erste arterielle Phase, erste portalvenöse Phase und erste Spätphase bezeichnet.

Es ist denkbar, dass mehrere MRT-Aufnahmen während der ersten hepatobiliären Phase erzeugt werden.

Nach der Erzeugung einer oder mehrerer erster MRT-Aufnahmen während der ersten hepatobiliären Phase wird ein zweites Mal Kontrastmittel appliziert. Beim zweiten Mal wird ein zweites Kontrastmittel appliziert. Bei dem zweiten Kontrastmittel kann es sich um das gleiche Kontrastmittel handeln wie das erste Kontrastmittel, es kann sich bei dem zweiten Kontrastmittel aber auch um ein anderes, vorzugsweise ein extrazelluläres Kontrastmittel handeln. Das zweite Kontrastmittel wird ebenfalls vorzugsweise intravenös als Bolus, gewichtsadaptiert verabreicht (z.B. in eine Armvene).

Die Applikation des ersten Kontrastmittels wird in dieser Beschreibung auch als erste Applikation bezeichnet; die Applikation des zweiten Kontrastmittels wird in dieser Beschreibung auch als zweite Applikation bezeichnet. Sind das erste Kontrastmittel und das zweite Kontrastmittel gleich, dann erfolgt also eine erste Applikation eines hepatobiliären Kontrastmittels und zu einem späteren Zeitpunkt eine zweite Applikation des hepatobiliären Kontrastmittels. Sind das erste Kontrastmittel und das zweite Kontrastmittel verschieden, erfolgt eine erste Applikation eines ersten Kontrastmittels, wobei es sich bei dem ersten Kontrastmittel um ein hepatobiliäres Kontrastmittel handelt, und es erfolgt zu einem späteren Zeitpunkt eine zweite Applikation eines zweiten (anderen) Kontrastmittels.

Zum Zeitpunkt der zweiten Applikation (bzw. zum Zeitpunkt der Applikation des zweiten Kontrastmittels) befindet sich das Untersuchungsobjekt vorzugsweise bereits in dem Kernspintomographen. Nach der Applikation des zweiten Kontrastmittels wird wiederum eine arterielle Phase, eine portalvenöse Phase und eine Spätphase durchlaufen. Diese arterielle Phase, portalvenöse Phase und Spätphase werden in dieser Beschreibung auch als zweite arterielle Phase, zweite portalvenöse Phase und zweite Spätphase bezeichnet. In der zweiten arteriellen Phase und/oder in der zweiten portalvenösen Phase und/oder in der zweiten Spätphase wird eine MRT-Aufhahme oder es werden mehrere MRT-Aufnahmen erzeugt. Diese MRT-Aufhahmen werden in der Reihenfolge ihrer Aufnahme als zweite, dritte, vierte usw. bezeichnet.

In einer bevorzugten Ausführungsform wird eine zweite MRT-Aufnahme während der zweiten arteriellen Phase, eine dritte MRT-Aufnahme während der zweiten portalvenösen Phase und eine vierte MRT-Aufhahme während der zweiten Spätphase erzeugt. Eine solche zweite MRT-Aufnahme zeigt insbesondere Arterien kontrastverstärkt; eine solche dritte MRT-Aufnahme zeigt insbesondere Venen kontrastverstärkt.

Denkbar ist ferner, dass während der genannten Phasen mehr als eine MRT-Aufnahmε erzeugt werden.

Die gemessenen MRT-Aufnahmen können als zweidimensionale Bildaufnahmen vorliegen, die eine Schnittebene durch das Untersuchungsobjekt zeigen. Die gemessenen MRT-Aufhahmen können als Stapel zweidimensionaler Bildaufnahme vorliegen, wobei jede einzelne Bildaufnahme des Stapels eine andere Schnittebene zeigt. Die gemessenen MRT-Aufnahmen können als dreidimensionale Aufnahmen (3D-Aufhahmen) vorliegen. Aus Gründen der einfacheren Darstellung wird die Erfindung an einigen Stellen der vorliegenden Beschreibung anhand des Vorliegens von zweidimensionalen MRT-Aufnahmen erläutert, ohne die Erfindung jedoch auf zweidimensionale MRT-Aufnahmen beschränken zu wollen. Dem Fachmann ist klar, wie sich das jeweils Beschriebene auf Stapel zweidimensionaler Bildaufhahmen und auf 3D-Aufnahmen übertragen lässt (siehe hierzu z.B. M. Reisler, W. Semmler: Magnetresonanztomographie, Springer Verlag, 3. Auflage, 2002, ISBN: 978-3-642-63076-7).

Üblicherweise liegen die gemessenen MRT-Aufnahmen als digitale Bilddateien vor. Der Begriff "digital" bedeutet, dass die MRT-Aufnahmen von einer Maschine, in der Regel einem Computersystem, verarbeitet werden können. Unter "Verarbeitung" werden die bekannten Verfahren zur elektronischen Datenverarbeitung (EDV) verstanden.

Digitale Bilddateien können in verschiedenen Formaten vorliegen. Digitale Bilddateien können beispielsweise als Rastergrafiken kodiert werden. Rastergrafiken bestehen aus einer rasterförmigen Anordnung von so genannten Bildpunkten (Pixel) oder Volumenelementen (Voxel), denen jeweils eine Farbe bzw. ein Grauwert zugeordnet ist. Die Hauptmerkmale einer 2D-Rastergrafik sind daher die Bildgröße (Breite und Höhe gemessen in Pixeln, umgangssprachlich auch Bildauflösung genannt) sowie die Farbtiefe. Einem Bildpunkt einer digitalen Bilddatei ist üblicherweise eine Farbe zugeordnet. Die für einen Bildpunkt verwendete Kodierung der Farbe definiert sich unter anderem über den Farbraum und die Farbtiefe. Der einfachste Fall ist ein Binärbild, bei dem ein Bildpunkt einen Schwarzweiß-Wert speichert. Bei einem Bild, dessen Farbe über den so genannten RGB-Farbraum definiert ist (RGB steht für die Grundfarben Rot, Grün und Blau), besteht jeder Bildpunkt aus drei Subpixeln, einem Subpixel für die Farbe Rot, einem Subpixel für die Farbe Grün und einem Subpixel für die Farbe Blau. Die Farbe eines Bildpunktes ergibt durch die Überlagerung (additives Mischen) der Farbwerte der Subpixel. Der Farbwert eines Subpixels ist in 256 Farbnuancen unterteilt, die Tonwerte genannt werden und üblicherweise von 0 bis 255 reichen. Die Farbnuance "0" eines jeden Farbkanals ist die dunkelste. Haben alle drei Kanäle den Tonwert 0, erscheint der entsprechende Bildpunkt schwarz; haben alle drei Kanäle den Tonwert 255, erscheint der entsprechende Bildpunkt weiß. Bei der Ausführung der vorliegenden Erfindung werden digitale Bilddateien (MRT-Aufnahmen) bestimmten Operationen unterworfen. Die Operationen betreffen dabei überwiegend die Bildpunkte, bzw. die Tonwerte der einzelnen Bildpunkte. Es gibt eine Vielzahl an möglichen digitalen Bildformaten und Farbkodierungen. Vereinfachend wird in dieser Beschreibung davon ausgegangen, dass die vorliegenden Bilder Graustufen-Rastergrafiken mit einer spezifischen Zahl an Bildpunkten sind, wobei jedem Bildpunkt ein Tonwert zugeordnet ist, der den Grauwert des Bildes angibt. Diese Annahme soll jedoch in keiner Weise limitierend verstanden werden. Dem Fachmann der Bildverarbeitung ist klar, wie er die Lehre dieser Beschreibung auf Bilddateien, die in anderen Bildformaten vorliegen und/oder bei denen die Farbwerte anders kodiert sind, übertragen kann.

Aus den MRT-Aufnahmen, die während einer oder mehrerer Phasen nach der Applikation des ersten und des zweiten Kontrastmittels erzeugt wurden, werden in einem nächsten Schritt MRT-Bilder erzeugt.

Dabei handelt es sich bei dem Begriff "MRT-Bild" um eine Sprachschöpfung. Der Begriff wurde erzeugt, um die verschiedenen im Rahmen der Erfindung erzeugten und verwendeten MRT-Aufnahmen besser auseinander halten zu können. Während es sich bei einer ersten, zweiten, dritten und vierten MRT-Aufnahme im Sinne der vorliegenden Erfindung um eine messtechnisch erzeugte Aufnahme handelt, wird unter einem MRT-Bild eine künstliche Darstellung verstanden, die das Ergebnis von Berechnungen ist. Ein MRT-Bild wird also selbst nicht messtechnisch erzeugt, sondern es wird aus mindestens zwei messtechnisch erzeugten MRT-Aufnahmen berechnet. MRT-Bilder liegen vorzugsweise in derselben Form vor wie die MRT-Aufnahmen, aus denen sie erzeugt werden (z.B. in Form digitaler Bilddateien).

Aus jeder MRT-Aufnahme, die während einer oder mehrerer Phasen nach der Applikation des zweiten Kontrastmittels erzeugt wurde, wird vorzugsweise unter Verwendung der ersten MRT-Aufhahme jeweils ein MRT-Bild berechnet: aus der zweiten MRT-Aufnahme und vorzugsweise der ersten MRT-Aufnahme wird ein erstes MRT-Bild berechnet; wenn vorhanden wird aus der dritten MRT-Aufnahme und vorzugsweise der ersten MRT-Aufnahme ein zweites MRT-Bild berechnet; wenn vorhanden wird aus der vierten MRT-Aufnahme und vorzugsweise der ersten MRT-Aufnahme ein drittes MRT-Bild berechnet; und so fort.

Das Ziel der Erzeugung von MRT-Bildem aus den MRT-Aufnahmen ist, den Kontrast zwischen gesundem Lebergewebe und anderen Bereichen zu erhöhen. Bei Verwendung eines hepatobiliären paramagnetischen Kontrastmittels als erstes Kontrastmittel ist die Signalintensität von gesundem Lebergewebe während der zweiten arteriellen Phase, der zweiten portalvenösen Phase und der zweiten Spätphase noch infolge der Applikation des ersten Kontrastmittels erhöht. Das zweite Kontrastmittel, das sich in den genannten zweiten Phasen ausbreitet, führt in dem Gewebe, in dem es sich ausbreitet, ebenfalls zu einem erhöhten Signal. Damit besteht in den MRT-Aufnahmen zwischen dem gesunden Lebergewebe und dem übrigen durch (zweites) Kontrastmittel kontrastverstärkten Gewebe nur ein geringer Kontrast. Um diesen Kontrast zu erhöhen, wird in einer bevorzugten Ausführungsform die erste MRT-Aufnahme von der zweiten MRT-Aufnahme subtrahiert. Falls vorhanden kann die erste MRT-Aufnahme auch von der dritten MRT-Aufnahme subtrahiert werden. Falls vorhanden kann die erste MRT-Aufnahme auch von der vierten MRT-Aufnahme subtrahiert werden. Und so fort.

Bei einer solchen Subtraktion werden üblicherweise die Tonwerte korrespondierender Pixel oder Voxel voneinander abgezogen. Zwei Pixel oder Voxel korrespondieren dann miteinander, wenn sie denselben Untersuchungsbereich zeigen.

Es ist denkbar, dass die MRT-Aufnahmen, die einer Subtraktion unterzogen werden, vorher einer Bewegungskorrektur unterzogen werden. Eine solche Bewegungskorrektur sorgt dafür, dass ein Pixel oder Voxel der ersten MRT-Aufnahme denselben Untersuchungsbereich zeigt, wie das entsprechende Pixel oder Voxel der zweiten MRT-Aufnahme, der dritten MRT-Aufnahme, der vierten MRT-Aufnahme und so weiter. Bewegungskorrekturverfahren sind im Stand der Technik beschrieben (siehe zum Beispiel: EP3118644, EP3322997, US20080317315, US20170269182, US20140062481, EP2626718).

Durch die Subtraktion von MRT-Aufnahmen wird insbesondere die Signalintensität bei dem gesunden Lebergewebe reduziert.

Die MRT-Bilder, die bei der Subtraktion der ersten MRT-Aufnahme von der zweiten MRT-Aufnahme und von der dritten MRT-Aufnahme und von der vierten MRT-Aufhahme und so weiter erzeugt werden, zeigen die Verteilung von Kontrastmittel in dem Gewebe näherungsweise so wie sie sein würde, wenn nur Kontrastmittel in Form eines einzigen Bolus appliziert worden wäre - mit dem Unterschied, dass die MRT-Untersuchung verkürzt ist, da das Untersuchungsobjekt eine kürzere Zeitspanne in dem Kernspintomographen verbringen muss.

In einer weiteren Ausführungsform erfolgt die Berechnung eines oder mehrerer MRT-Bilder mit Hilfe eines künstlichen neuronalen Netzes, das trainiert ist, aus einer ersten MRT-Aufnahme und einer zweiten MRT-Aufnahme ein MRT-Bild zu erzeugen. Die erste MRT-Aufhahme zeigt eine Leber oder einen Teil einer Leber eines Untersuchungsobjekts in einer ersten hepatobiliären Phase, d.h. zu einem ersten Zeitpunkt nach einer Applikation eines hepatobiliären Kontrastmittels, wobei gesunde Leberzellen zu dem ersten Zeitpunkt infolge der Applikation des ersten Kontrastmittels in der ersten MRT-Aufnahme kontrastverstärkt dargestellt sind. Die zweite MRT-Aufnahme zeigt dieselbe Leber oder denselben Teil der Leber zu einem zweiten Zeitpunkt nach einer Applikation eines zweiten Kontrastmittels, wobei gesunde Leberzellen zu dem zweiten Zeitpunkt infolge der Applikation des ersten Kontrastmittels in der zweiten MRT-Aufnahme kontrastverstärkt dargestellt sind, und wobei Blutgefäße zu dem zweiten Zeitpunkt infolge der Applikation des zweiten Kontrastmittels in der zweiten MRT-Aufnahme kontrastverstärkt dargestellt sind. Das MRT-Bild zeigt dieselbe Leber oder denselben Teil der Leber so wie sie/er aussehen würde, wenn nur das zweite Kontrastmittel appliziert worden wäre: die Blutgefäße sind infolge der Applikation des zweiten Kontrastmittels kontrastverstärkt dargestellt, aber gesunde Leberzellen sind nicht infolge der Applikation eines ersten Kontrastmittels kontrastverstärkt dargestellt. Mit anderen Worten, es wird ein MRT-Bild erzeugt, dass so aussieht wie die zweite MRT-Aufnahme, mit dem Unterschied, dass die Kontrastverstärkung der gesunden Leberzellen, die durch die Applikation des ersten Kontrastmittels verursacht worden ist, aus der zweiten MRT-Aufnahme herausgerechnet (eliminiert) wird.

Ein solches künstliches neuronales Netzwerk umfasst mindestens drei Schichten von Verarbeitungselementen: eine erste Schicht mit Eingangsneuronen (Knoten), eine N-te Schicht mit mindestens einem Ausgangsneuron (Knoten) und N-2 innere Schichten, wobei N eine natürliche Zahl und größer als 2 ist.

Die Eingangsneuronen dienen zum Empfangen von digitalen MRT-Aufnahmen als Eingangswerte, z.B. einer ersten MRT-Aufnahme und einer zweiten MRT-Aufnahme. Normalerweise gibt es ein Eingangsneuron für jedes Pixel oder Voxel einer digitalen MRT-Aufnahme. Es können zusätzliche Eingangsneuronen für zusätzliche Eingangswerte (z.B. Informationen zum Untersuchungsbereich, zum Untersuchungsobjekt, zu Bedingungen, die bei der Erzeugung der MRT-Aufhahmen herrschten, und/oder Informationen zu den Zeitpunkten oder Zeitspannen zu/in denen die MRT-Aufnahmen erzeugt worden sind) vorhanden sein.

In einem solchen Netzwerk dienen die Ausgangsneuronen dazu, ein MRT-Bild auszugeben.

Die Verarbeitungselemente der Schichten zwischen den Eingangsneuronen und den Ausgangsneuronen sind in einem vorbestimmten Muster mit vorbestimmten Verbindungsgewichten miteinander verbunden.

Vorzugsweise handelt es sich bei dem künstlichen neuronalen Netz um ein so genanntes Convolutional Neural Network (kurz: CNN).

Ein Convolutional Neural Network ist in der Lage, Eingabedaten in Form einer Matrix zu verarbeiten. Dies ermöglicht es, als Matrix dargestellte digitale MRT-Aufnahmen (z.B. Breite x Höhe x Farbkanäle) als Eingabedaten zu verwenden. Ein normales neuronales Netz z.B. in Form eines Multi-Layer-Perceptrons (MLP) benötigt dagegen einen Vektor als Eingabe, d.h. um eine MRT-Aufnahme als Eingabe zu verwenden, müssten die Pixel oder Voxel der MRT-Aufnahme in einer langen Kette hintereinander ausgerollt werden. Dadurch sind normale neuronale Netze z.B. nicht in der Lage, Objekte in einer MRT-Aufnahme unabhängig von der Position des Objekts in der MRT-Aufhahme zu erkennen. Das gleiche Objekt an einer anderen Position in der MRT-Aufhahme hätte einen völlig anderen Eingabevektor.

Ein CNN besteht im Wesentlichen aus Filtern (Convolutional Layer) und Aggregations-Schichten (Pooling Layer), die sich abwechselnd wiederholen, und am Ende aus einer Schicht oder mehreren Schichten von "normalen" vollständig verbundenen Neuronen (Dense / Fully Connected Layer).

Details sind dem Stand der Technik zu entnehmen (siehe z.B.: S. Khan et al.: A Guide to Convolutional Neural Networks for Computer Vision, Morgan & Claypool Publishers 2018, ISBN 1681730227, 9781681730226, WO2018/183044A1, WO2018/200493, WO2019/074938A1, WO2019/204406A1, WO2019/241659A1).

Das Trainieren des neuronalen Netzes kann in einem überwachten maschinellen Lernen mit einem Trainingsdatensatz erfolgen. Der Trainingsdatensatz umfasst eine Vielzahl an ersten Referenz-MRT-Aufnahmen, zweiten Referenz-MRT-Aufnahmen und dritten Referenz-MRT-Aufnahmen.

Eine solche erste Referenz-MRT-Aufnahme entspricht einer ersten MRT-Aufnahme: sie zeigt eine Leber oder einen Teil einer Leber eines Untersuchungsobjekts zu einem ersten Zeitpunkt nach einer Applikation eines ersten Kontrastmittels, wobei gesunde Leberzellen zu dem ersten Zeitpunkt infolge der Applikation des ersten Kontrastmittels in der ersten MRT-Aufnahme kontrastverstärkt dargestellt sind.

Eine solche zweite Referenz-MRT-Aufnalime entspricht einer zweiten MRT-Aufnahme: sie zeigt dieselbe Leber oder denselben Teil der Leber zu einem zweiten Zeitpunkt nach einer Applikation eines zweiten Kontrastmittels zeigt, wobei gesunde Leberzellen zu dem zweiten Zeitpunkt infolge der Applikation des ersten Kontrastmittels in der zweiten MRT-Aufnahme kontrastverstärkt dargestellt sind, und wobei Blutgefäße zu dem zweiten Zeitpunkt infolge der Applikation des zweiten Kontrastmittels in der zweiten MRT-Aufnahme kontrastverstärkt dargestellt sind. Bei den Blutgefäßen kann es sich um Arterien und/oder Venen handeln.

Eine solche dritte Referenz-MRT-Aufnahme zeigt dieselbe Leber oder denselben Teil der Leber zu einem zweiten Zeitpunkt nach einer Applikation eines zweiten Kontrastmittels ohne dass vor dem zweiten Zeitpunkt ein erstes Kontrastmittel appliziert worden ist: nur Blutgefäße sind zu dem zweiten Zeitpunkt infolge der Applikation des zweiten Kontrastmittels in der zweiten MRT-Aufnahme kontrastverstärkt dargestellt.

Beim maschinellen Lernen wird ein statistisches Modell erzeugt, das auf den Trainingsdaten beruht. Das heißt, es werden nicht einfach die Beispiele auswendig gelernt, sondern das Modell "erkennt" Muster und Gesetzmäßigkeiten in den Trainingsdaten. So kann das Modell auch unbekannte Daten beurteilen. Validierungsdaten können verwendet werden, um die Güte der Beurteilung unbekannter Daten zu prüfen.

Das Trainieren des Modells erfolgt üblicherweise mittels überwachten Lernens (engl.: *supervised learning*), d.h. dem Modell werden erste und zweite Referenz-MRT-Aufnahmen präsentiert und es wird ihm mitgeteilt, welche dritten Referenz-MRT-Aufnahmen mit den jeweiligen ersten und zweiten Referenz-MRT-Aufhahmen verbunden sind. Der Algorithmus lernt dann eine Beziehung zwischen den Referenz-MRT-Aufnahmen, um für unbekannte erste und zweite MRT-Aufnahmen dritte MRT-Bilder vorherzusagen (zu berechnen).

Selbstlernende Algorithmen, die mittels überwachten Lernens trainiert werden, sind vielfältig im Stand der Technik beschrieben (siehe z.B. C. Perez: Machine Learning Techniques: Supervised Learning and Classification, Amazon Digital Services LLC - Kdp Print Us, 2019, ISBN 1096996545, 9781096996545, WO2018/183044A1, WO2018/200493, WO2019/074938A1, WO2019/204406A1, WO2019/241659A1).

Das Trainieren des neuronalen Netzes kann beispielsweise mittels eines Backpropagation-Verfahrens durchgeführt werden. Dabei wird für das Netz eine möglichst zuverlässige Abbildung von gegebenen Eingabedaten auf gegebene Ausgabedaten angestrebt. Die Qualität der Abbildung wird durch eine Fehlerfunktion beschrieben. Das Ziel ist die Minimierung der Fehlerfunktion. Das Einlernen eines künstlichen neuronalen Netzes erfolgt bei dem Backpropagation-Verfahren durch die Änderung der Verbindungsgewichte.

Im trainierten Zustand enthalten die Verbindungsgewichte zwischen den Verarbeitungselementen Informationen bezüglich der Beziehung zwischen den ersten und zweiten Referenz-MRT-Aufnahmen und den dritten Referenz-MRT-Aufnahmen, die verwendet werden können, µm für neue erste und zweite MRT-Aufnahme die entsprechenden MRT-Bilder vorherzusagen.

Eine Kreuzvalidierungsmethode kann verwendet werden, um die Daten in Trainings- und Validierungsdatensätze aufzuteilen. Der Trainingsdatensatz wird beim Backpropagation-Training der Netzwerkgewichte verwendet. Der Validierungsdatensatz wird verwendet, um zu überprüfen, mit welcher Vorhersagegenauigkeit sich das trainierte Netzwerk auf unbekannte MRT-Aufnahmen anwenden lässt.

Wie bereits beschrieben, können zum Trainieren, Validieren und Vorhersagen auch weitere Informationen zum Untersuchungsobjekt, zum Untersuchungsbereich, zu Untersuchungsbedingungen und/oder zu den gemessenen MRT-Aufnahmen verwendet werden.

Beispiele für Informationen zum Untersuchungsobjekt sind: Geschlecht, Alter, Gewicht, Größe, Anamnese, Art und Dauer und Menge bereits eingenommener Medikamente, Blutdruck, zentralvenöser Druck, Atemfrequenz, Serum Albumin, Total Bilirubin, Blutzucker, Eisengehalt, Atemkapazität und dergleichen. Diese können z.B. auch einer Datenbank bzw. einer elektronischen Patientenakte herangezogen werden.

Beispiele für Informationen zum Untersuchungsbereich sind: Vorerkrankungen, Operationen, Teilresektion, Lebertransplantation, Eisenleber, Fettleber und dergleichen.

Es ist denkbar, dass MRT-Aufnahmen einer Bewegungskorrektur unterzogen werden, bevor sie dem Vorhersagemodell zugeführt werden. Eine solche Bewegungskorrektur sorgt dafür, dass ein Pixel oder Voxel einer ersten MRT-Aufnahme denselben Untersuchungsbereich zeigt, wie das entsprechende Pixel oder Voxel einer zweiten MRT-Aufnahme. Bewegungskorrekturverfahren sind im Stand der Technik beschrieben (siehe zum Beispiel: EP3118644, EP3322997, US20080317315, US20170269182, US20140062481, EP2626718).

Ein Gegenstand der vorliegenden Erfindung ist ein System, mit dem das erfindungsgemäße Verfahren ausgeführt werden kann.

Ein solches System weist eine Empfangseinheit, eine Steuer- und Recheneinheit und eine Ausgabeeinheit auf.

Es ist denkbar, dass die genannten Einheiten Bestandteile eines einzigen Computersystems sind; es ist aber auch denkbar, dass die genannten Einheiten Bestandteile von mehreren separaten Computersystemen sind, die über ein Netzwerk miteinander verbunden sind, um Daten und/oder Steuersignale von einer Einheit zu einer anderen Einheit zu übermitteln.

Ein "Computersystem" ist ein System zur elektronischen Datenverarbeitung, das mittels programmierbarer Rechenvorschriften Daten verarbeitet. Ein solches System umfasst üblicherweise einen "Computer", diejenige Einheit, die einen Prozessor zur Durchführung logischer Operationen umfasst, sowie eine Peripherie.

Als "Peripherie" bezeichnet man in der Computertechnik alle Geräte, die an den Computer angeschlossen sind, und zur Steuerung des Computers und/oder als Ein- und Ausgabegeräte dienen. Beispiele hierfür sind Monitor (Bildschirm), Drucker, Scanner, Maus, Tastatur, Laufwerke, Kamera, Mikrofon, Lautsprecher etc. Auch interne Anschlüsse und Erweiterungskarten gelten in der Computertechnik als Peripherie.

Computersysteme von heute werden häufig in Desktop-PCs, Portable PCs, Laptops, Notebooks, Netbooks und Tablet-PCs und so genannte Handhelds (z.B. Smartphone) unterteilt; alle diese Systeme können zur Ausführung der Erfindung genutzt werden.

Eingaben in das Computersystem erfolgen über Eingabemittel wie beispielsweise eine Tastatur, eine Maus, ein Mikrofon, ein berührungsempfindliches Display und/oder dergleichen.

Das erfindungsgemäße System ist konfiguriert, aus jeweils zwei MRT-Aufnahmen jeweils ein MRT-Bild zu erzeugen. Vorzugsweise erfolgt dies durch Subtraktion der Tonwerte korrespondierender Bildpunkte der zwei MRT-Aufnahmen. Dabei ist es denkbar, dass die absolute Differenz gebildet wird, um negative Tonwerte zu vermeiden. Ferner ist denkbar, dass ein negativer Tonwert auf den Tonwert Null gesetzt wird, um negative Tonwerte zu vermeiden.

Die Empfangseinheit dient dem Empfang von MRT-Aufnahmen. Die MRT-Aufnahmen können beispielsweise von einer Magnetresonanzanlage übermittelt werden oder aus einem Datenspeicher ausgelesen werden. Die Magnetresonanzanlage kann ein Bestandteil des erfindungsgemäßen Systems sein. Denkbar ist aber auch, dass das erfindungsgemäße System ein Bestandteil einer Magnetresonanzanlage ist.

Von der Empfangseinheit werden zumindest die erste MRT-Aufhahme und die zweite MRT-Aufnahme an die Steuer- und Recheneinheit übermittelt. Falls vorhanden können auch die dritte, vierte und so weiter MRT-Aufnahmen übermittelt werden.

Die Steuer- und Recheneinheit dient der Berechnung von MRT-Bildern aus den empfangenen MRT-Aufnahmen. Die Steuer- und Recheneinheit dient ferner der Steuerung der Empfangseinheit sowie der Koordinierung der Daten- und Signalflüsse zwischen verschiedenen Einheiten. Es ist denkbar, dass mehrere Steuer- und Recheneinheiten vorhanden sind.

Über die Ausgabeeinheit können die berechneten MRT-Bilder angezeigt werden (zum Beispiel auf einem Monitor), ausgegeben werden (z.B. über einen Drucker) oder in einem Datenspeicher gespeichert werden.

Weitere Ausführungsformen der Erfindung sind:
1. Ein Verfahren umfassend die Schritte
   - Empfangen einer ersten MRT-Aufnahme eines Untersuchungsobjekts, wobei die erste MRT-Aufnahme eine Leber oder einen Teil einer Leber des Untersuchungsobjekts zu einem ersten Zeitpunkt nach einer ersten Applikation eines Kontrastmittels zeigt, wobei gesunde Leberzellen zu dem ersten Zeitpunkt infolge der ersten Applikation des Kontrastmittels in der ersten MRT-Aufnahme kontrastverstärkt dargestellt sind,
   - Empfangen einer zweiten MRT-Aufnahme desselben Untersuchungsobjekts, wobei die zweite MRT-Aufnahme dieselbe Leber oder denselben Teil der Leber zu einem zweiten Zeitpunkt nach einer zweiten Applikation des Kontrastmittels zeigt, wobei gesunde Leberzellen zu dem zweiten Zeitpunkt infolge der ersten Applikation des Kontrastmittels in der zweiten MRT-Aufnahme kontrastverstärkt dargestellt sind, und wobei Blutgefäße zu dem zweiten Zeitpunkt infolge der zweiten Applikation des Kontrastmittels in der zweiten MRT-Aufnahme kontrastverstärkt dargestellt sind,
   - Erzeugen eines ersten MRT-Bildes aus der ersten MRT-Aufnahme und der zweiten MRT-Aufnahme, wobei das erste MRT-Bild dieselbe Leber oder denselben Teil der Leber zu dem zweiten Zeitpunkt zeigt, wobei der Kontrast zwischen gesunden Leberzellen und Blutgefäßen in dem ersten MRT-Bild gegenüber der zweiten MRT-Aufnahme vergrößert ist,
   - Anzeigen und/oder Ausgeben des ersten MRT-Bildes und/oder Speichern des ersten MRT-Bildes in einem Datenspeicher.
2. Das Verfahren gemäß der Ausführungsform 1, umfassend die Schritte
   - Empfangen der ersten MRT-Aufnahme des Untersuchungsobjekts, wobei die erste MRT-Aufnahme die Leber oder den Teil einer Leber des Untersuchungsobjekts zu dem ersten Zeitpunkt nach der ersten Applikation des Kontrastmittels während einer ersten hepatobiliären Phase zeigt,
   - Empfangen der zweiten MRT-Aufnahme desselben Untersuchungsobjekts, wobei die zweite MRT-Aufnahme dieselbe Leber oder denselben Teil der Leber zu dem zweiten Zeitpunkt nach der zweiten Applikation des Kontrastmittels während einer zweiten arteriellen Phase zeigt,
   - Empfangen eier dritten MRT-Aufnahme desselben Untersuchungsobjekts, wobei die dritte MRT-Aufnahme dieselbe Leber oder denselben Teil der Leber zu einem dritten Zeitpunkt nach der zweiten Applikation des Kontrastmittels während einer zweiten portalvenösen Phase zeigt,
   - Empfangen einer vierten MRT-Aufnahme desselben Untersuchungsobjekts, wobei die vierte MRT-Aufnahme dieselbe Leber oder denselben Teil der Leber zu einem vierten Zeitpunkt nach der zweiten Applikation des Kontrastmittels während einer zweiten Spätphase zeigt,
   - Erzeugen von MRT-Bildern aus den empfangenen MRT-Aufnahmen, wobei das erste MRT-Bild durch Subtraktion der ersten MRT-Aufnahme von der zweiten MRT-Aufnahme berechnet wird, wobei ein zweites MRT-Bild durch Subtraktion der ersten MRT-Aufnahme von der dritten MRT-Aufnahme berechnet wird, wobei ein drittes MRT-Bild durch Subtraktion der ersten MRT-Aufnahme von der dritten MRT-Aufnahme berechnet wird,
   - Anzeigen und/oder Ausgeben eines oder mehrerer der erzeugten MRT-Bilder und/oder Speichern eines oder mehrerer der erzeugten MRT-Bilder in einem Datenspeicher.
3. Das Verfahren gemäß einer der Ausführungsformen 1 oder 2, wobei es sich bei den MRT-Aufnahmen um T1-gewichtete Darstellungen der Leber oder des Teils der Leber nach Applikation eines hepatobiliären, paramagnetischen Kontrastmittels handelt.
4. Das Verfahren gemäß einer der Ausführungsformen 1 bis 3, wobei es sich bei dem Kontrastmittel um einen Stoff oder ein Stoffgemisch mit Gadoxetsäure oder einem Salz der Gadoxetsäure als kontrastverstärkenden Wirkstoff, bevorzugt um das Dinatriumsalz der Gadoxetsäure handelt.
5. Das Verfahren gemäß einer der Ausführungsformen 1 bis 4, wobei es sich beim Untersuchungsobjekt um ein Säugetier, vorzugsweise um einen Menschen handelt.
6. Ein System umfassend
   - eine Empfangseinheit,
   - eine Steuer- und Recheneinheit und
   - eine Ausgabeeinheit,
      - wobei die Steuer- und Recheneinheit konfiguriert ist, die Empfangseinheit zu veranlassen, eine erste MRT-Aufhahme eines Untersuchungsobjekts zu empfangen, wobei die erste MRT-Aufnahme eine Leber oder einen Teil einer Leber des Untersuchungsobjekts zu einem ersten Zeitpunkt nach einer ersten Applikation eines Kontrastmittels zeigt, wobei gesunde Leberzellen zu dem ersten Zeitpunkt infolge der ersten Applikation des Kontrastmittels in der ersten MRT-Aufnahme kontrastverstärkt dargestellt sind,
      - wobei die Steuer- und Recheneinheit konfiguriert ist, die Empfangseinheit zu veranlassen, eine zweite MRT-Aufnahme eines Untersuchungsobjekts zu empfangen, wobei die zweite MRT-Aufnahme dieselbe Leber oder denselben Teil der Leber zu einem zweiten Zeitpunkt nach einer zweiten Applikation des Kontrastmittels zeigt, wobei gesunde Leberzellen zu dem zweiten Zeitpunkt infolge der ersten Applikation des Kontrastmittels in der zweiten MRT-Aufnahme kontrastverstärkt dargestellt sind, und wobei Blutgefäße zu dem zweiten Zeitpunkt infolge der zweiten Applikation des Kontrastmittels in der zweiten MRT-Aufnahme kontrastverstärkt dargestellt sind,
      - wobei die Steuer- und Recheneinheit konfiguriert ist, aus der ersten MRT-Aufnahme und der zweiten MRT-Aufnahme ein erstes MRT-Bild zu erzeugen, wobei das erste MRT-Bild dieselbe Leber oder denselben Teil der Leber zu dem zweiten Zeitpunkt zeigt, wobei der Kontrast zwischen gesunden Leberzellen und Blutgefäßen in dem ersten MRT-Bild gegenüber der zweiten MRT-Aufnahmε vergrößert ist,
      - wobei die Steuer- und Recheneinheit konfiguriert ist, die Ausgabeeinheit zu veranlassen, das erste MRT-Bild anzuzeigen, auszugeben oder in einem Datenspeicher zu speichern.
7. Das Computersystem gemäß der Ausführungsform 6,
   - wobei die Steuer- und Recheneinheit konfiguriert ist, die Empfangseinheit zu veranlassen, eine erste MRT-Aufnahme, eine zweite MRT-Aufnahme, eine dritte MRT-Aufnahme und eine vierte MRT-Aufhahme zu empfangen,
      - wobei die erste MRT-Aufnahme die Leber oder den Teil der Leber des Untersuchungsobjekts zu dem ersten Zeitpunkt nach der ersten Applikation des Kontrastmittels während einer ersten hepatobiliären Phase zeigt,
      - wobei die zweite MRT-Aufnahme dieselbe Leber oder denselben Teil der Leber zu einem zweiten Zeitpunkt nach der zweiten Applikation des Kontrastmittels während einer zweiten arteriellen Phase zeigt,
      - wobei die dritte MRT-Aufhahme dieselbe Leber oder denselben Teil der Leber zu einem dritten Zeitpunkt nach der zweiten Applikation des Kontrastmittels während einer zweiten portalvenösen Phase zeigt,
      - wobei die vierte MRT-Aufnahme dieselbe Leber oder denselben Teil der Leber zu einem vierten Zeitpunkt nach der zweiten Applikation des Kontrastmittels während einer zweiten Spätphase zeigt,
   - wobei die Steuer- und Recheneinheit konfiguriert ist, MRT-Bilder aus den empfangenen MRT-Aufnahmen zu berechnen, wobei ein erstes MRT-Bild durch Subtraktion der ersten MRT-Aufnahme von der zweiten MRT-Aufnahme berechnet wird, wobei ein zweites MRT-Bild durch Subtraktion der ersten MRT-Aufnahme von der dritten MRT-Aufnahme berechnet wird, wobei ein drittes MRT-Bild durch Subtraktion der ersten MRT-Aufnahme von der dritten MRT-Aufnahme berechnet wird,
   - wobei die Steuer- und Recheneinheit konfiguriert ist, die Ausgabeeinheit zu veranlassen, ein oder mehrere der berechneten MRT-Bilder auf einem Bildschirm anzuzeigen und/oder auf einem Drucker auszugeben und/oder in einem Datenspeicher zu speichern.
8. Ein Computerprogrammprodukt umfassend ein Computerprogramm, das in einen Arbeitsspeicher eines Computers geladen werden kann und dort den Computer dazu veranlasst, folgende Schritte ausführen:
   - Empfangen einer ersten MRT-Aufnahme eines Untersuchungsobjekts, wobei die erste MRT-Aufnahme eine Leber oder einen Teil einer Leber des Untersuchungsobjekts zu einem ersten Zeitpunkt nach einer ersten Applikation eines Kontrastmittels zeigt, wobei gesunde Leberzellen zu dem ersten Zeitpunkt infolge der ersten Applikation des Kontrastmittels in der ersten MRT-Aufnahme kontrastverstärkt dargestellt sind,
   - Empfangen einer zweiten MRT-Aufnahme, wobei die zweite MRT-Aufnahme dieselbe Leber oder denselben Teil der Leber zu einem zweiten Zeitpunkt nach einer zweiten Applikation des Kontrastmittels zeigt, wobei gesunde Leberzellen zu dem zweiten Zeitpunkt infolge der ersten Applikation des Kontrastmittels in der zweiten MRT-Aufnahmε kontrastverstärkt dargestellt sind, und wobei Blutgefäße zu dem zweiten Zeitpunkt infolge der zweiten Applikation des Kontrastmittels in der zweiten MRT-Aufnahme kontrastverstärkt dargestellt sind,
   - Erzeugen eines ersten MRT-Bildes aus der ersten MRT-Aufnahme und der zweiten MRT-Aufnahme, wobei das erste MRT-Bild dieselbe Leber oder denselben Teil der Leber zu dem zweiten Zeitpunkt zeigt, wobei der Kontrast zwischen gesunden Leberzellen und Blutgefäßen in dem ersten MRT-Bild gegenüber der zweiten MRT-Aufnahme vergrößert ist,
   - Anzeigen und/oder Ausgeben des ersten MRT-Bildes und/oder Speichern des ersten MRT-Bildes in einem Datenspeicher.
9. Das Computerprogrammprodukt gemäß der Ausführungsform 8, wobei das Computerprogramm den Computer dazu veranlasst die folgenden Schritte auszuführen:
   - Empfangen der ersten MRT-Aufhahme des Untersuchungsobjekts, wobei die erste MRT-Aufnahme die Leber oder den Teil der Leber des Untersuchungsobjekts zu dem ersten Zeitpunkt nach der ersten Applikation des Kontrastmittels während einer ersten hepatobiliären Phase zeigt,
   - Empfangen der zweiten MRT-Aufnahme, wobei die zweite MRT-Aufhahme dieselbe Leber oder denselben Teil der Leber zu dem zweiten Zeitpunkt nach der zweiten Applikation des Kontrastmittels während einer zweiten arteriellen Phase zeigt,
   - Empfangen einer dritten MRT-Aufnahme, wobei die dritte MRT-Aufhahme dieselbe Leber oder denselben Teil der Leber zu einem dritten Zeitpunkt nach der zweiten Applikation des Kontrastmittels während einer zweiten portalvenösen Phase zeigt,
   - Empfangen einer vierten MRT-Aufnahme, wobei die vierte MRT-Aufnahme dieselbe Leber oder denselben Teil der Leber zu einem vierten Zeitpunkt nach der zweiten Applikation des Kontrastmittels während einer zweiten Spätphase zeigt,
   - Erzeugen von MRT-Bildern aus den empfangenen MRT-Aufnahmen, wobei das erste MRT-Bild durch Subtraktion der ersten MRT-Aufnahme von der zweiten MRT-Aufnahme berechnet wird, wobei ein zweites MRT-Bild durch Subtraktion der ersten MRT-Aufnahme von der dritten MRT-Aufnahme berechnet wird, wobei ein drittes MRT-Bild durch Subtraktion der ersten MRT-Aufnahme von der dritten MRT-Aufnahme berechnet wird,
   - Anzeigen und/oder Ausgeben eines oder mehrerer der erzeugten MRT-Bilder und/oder Speichern eines oder mehrerer der erzeugten MRT-Bilder in einem Datenspeicher.
10. Das Computerprogrammprodukt gemäß der Ausführungsform 9, wobei das Computerprogramm den Computer dazu veranlasst, einen oder mehrere der in den Ansprüchen 1 bis 5 aufgeführten Schritte auszuführen.
11. Eine Verwendung eines Kontrastmittels in einem MRT-Verfahren, wobei das MRT-Verfahren die folgenden Schritte umfasst:
   - erstes Applizieren des Kontrastmittels,
   - Erzeugen einer ersten MRT-Aufnahmε, wobei die erste MRT-Aufnahme eine Leber oder einen Teil einer Leber zu einem ersten Zeitpunkt nach der ersten Applikation des Kontrastmittels
   zeigt, wobei gesunde Leberzellen zu dem ersten Zeitpunkt infolge der ersten Applikation des Kontrastmittels in der ersten MRT-Aufnahme kontrastverstärkt dargestellt sind,
   - zweites Applizieren des Kontrastmittels zu einem Zeitpunkt nach dem Erzeugen der ersten MRT-Aufnahme,
   - Erzeugen einer zweiten MRT-Aufnahme, wobei die zweite MRT-Aufnahme dieselbe Leber oder denselben Teil der Leber zu einem zweiten Zeitpunkt nach der zweiten Applikation des Kontrastmittels zeigt, wobei gesunde Leberzellen zu dem zweiten Zeitpunkt infolge der ersten Applikation des Kontrastmittels in der zweiten MRT-Aufnahme kontrastverstärkt dargestellt sind, und wobei Blutgefäße zu dem zweiten Zeitpunkt infolge der zweiten Applikation des Kontrastmittels in der zweiten MRT-Aufnahme kontrastverstärkt dargestellt sind,
   - Erzeugen eines ersten MRT-Bildes aus der ersten MRT-Aufnahme und der zweiten MRT-Aufnahme, wobei das erste MRT-Bild dieselbe Leber oder denselben Teil der Leber zu dem zweiten Zeitpunkt zeigt, wobei der Kontrast zwischen gesunden Leberzellen und Blutgefäßen in dem ersten MRT-Bild gegenüber der zweiten MRT-Aufnahme vergrößert ist,
   - Anzeigen und/oder Ausgeben eines oder mehrere der erzeugten MRT-Bilder und/oder Speichern eines oder mehrerer der erzeugten MRT-Bilder in einem Datenspeicher.
12. Die Verwendung gemäß der Ausführungsform 11, umfassend die Schritte:
   - erstes Applizieren des Kontrastmittels,
   - Erzeugen der ersten MRT-Aufnahme, wobei die erste MRT-Aufhahme die Leber oder den Teil der Leber des Untersuchungsobjekts zu dem ersten Zeitpunkt nach der ersten Applikation des Kontrastmittels während einer ersten hepatobiliären Phase zeigt,
   - zweites Applizieren des Kontrastmittels zu einem Zeitpunkt nach dem Erzeugen der ersten MRT-Aufnahme,
   - Erzeugen der zweiten MRT-Aufnahme, wobei die zweite MRT-Aufnahme dieselbe Leber oder denselben Teil der Leber zu dem zweiten Zeitpunkt nach der zweiten Applikation des Kontrastmittels während einer zweiten arteriellen Phase zeigt,
   - Erzeugen einer dritten MRT-Aufnahme, wobei die dritte MRT-Aufnahme dieselbe Leber oder denselben Teil der Leber zu einem dritten Zeitpunkt nach der zweiten Applikation des Kontrastmittels während einer zweiten portalvenösen Phase zeigt,
   - Erzeugen einer vierten MRT-Aufnahme, wobei die vierte MRT-Aufnahme dieselbe Leber oder denselben Teil der Leber zu einem vierten Zeitpunkt nach der zweiten Applikation des Kontrastmittels während einer zweiten Spätphase zeigt,
   - Erzeugen von MRT-Bildern aus den empfangenen MRT-Aufnahmen, wobei das erste MRT-Bild durch Subtraktion der ersten MRT-Aufnahme von der zweiten MRT-Aufnahme berechnet wird, wobei ein zweites MRT-Bild durch Subtraktion der ersten MRT-Aufhahme von der dritten MRT-Aufnahme berechnet wird, wobei ein drittes MRT-Bild durch Subtraktion der ersten MRT-Aufnahme von der dritten MRT-Aufnahme berechnet wird,
   - Anzeigen und/oder Ausgeben eines oder mehrerer der erzeugten MRT-Bilder und/oder Speichern eines oder mehrerer der erzeugten MRT-Bilder in einem Datenspeicher.
13. Ein Kontrastmittel zur Verwendung in einem MRT-Verfahren, wobei das MRT-Verfahren die folgenden Schritte umfasst:
   - erstes Applizieren des Kontrastmittels,
   - Erzeugen einer ersten MRT-Aufnahme, wobei die erste MRT-Aufnahme eine Leber oder einen Teil einer Leber zu einem ersten Zeitpunkt nach der ersten Applikation des Kontrastmittels zeigt, wobei gesunde Leberzellen zu dem ersten Zeitpunkt infolge der ersten Applikation des Kontrastmittels in der ersten MRT-Aufnahme kontrastverstärkt dargestellt sind,
   - zweites Applizieren des Kontrastmittels zu einem Zeitpunkt nach dem Erzeugen der ersten MRT-Aufnahme,
   - Erzeugen einer zweiten MRT-Aufnahme, wobei die zweite MRT-Aufnahme dieselbe Leber oder denselben Teil der Leber zu einem zweiten Zeitpunkt nach der zweiten Applikation des Kontrastmittels zeigt, wobei gesunde Leberzellen zu dem zweiten Zeitpunkt infolge der ersten Applikation des Kontrastmittels in der zweiten MRT-Aufnahme kontrastverstärkt dargestellt sind, und wobei Blutgefäße zu dem zweiten Zeitpunkt infolge der zweiten Applikation des Kontrastmittels in der zweiten MRT-Aufnahme kontrastverstärkt dargestellt sind,
   - Erzeugen eines ersten MRT-Bildes aus der ersten MRT-Aufnahme und der zweiten MRT-Aufnahme, wobei das erste MRT-Bild dieselbe Leber oder denselben Teil der Leber zu dem zweiten Zeitpunkt zeigt, wobei der Kontrast zwischen gesunden Leberzellen und Blutgefäßen in dem ersten MRT-Bild gegenüber der zweiten MRT-Aufhahme vergrößert ist,
   - Anzeigen und/oder Ausgeben eines oder mehrere der erzeugten MRT-Bilder und/oder Speichern eines oder mehrerer der erzeugten MRT-Bilder in einem Datenspeicher.
14. Ein Kontrastmittel zur Verwendung gemäß der Ausführungsform 13, wobei das MRT-Verfahren die folgenden Schritte umfasst:
   - erstes Applizieren des Kontrastmittels,
   - Erzeugen der ersten MRT-Aufnahme, wobei die erste MRT-Aufhahme die Leber oder den Teil der Leber des Untersuchungsobjekts zu dem ersten Zeitpunkt nach der ersten Applikation des Kontrastmittels während einer ersten hepatobiliären Phase zeigt,
   - zweites Applizieren des Kontrastmittels zu einem Zeitpunkt nach dem Erzeugen der ersten MRT-Aufnahme,
   - Erzeugen der zweiten MRT-Aufnahme, wobei die zweite MRT-Aufnahme dieselbe Leber oder denselben Teil der Leber zu dem zweiten Zeitpunkt nach der zweiten Applikation des Kontrastmittels während einer zweiten arteriellen Phase zeigt,
   - Erzeugen einer dritten MRT-Aufnahme, wobei die dritte MRT-Aufnahme dieselbe Leber oder denselben Teil der Leber zu einem dritten Zeitpunkt nach der zweiten Applikation des Kontrastmittels während einer zweiten portalvenösen Phase zeigt,
   - Erzeugen einer vierten MRT-Aufnahme, wobei die vierte MRT-Aufnahme dieselbe Leber oder denselben Teil der Leber zu einem vierten Zeitpunkt nach der zweiten Applikation des Kontrastmittels während einer zweiten Spätphase zeigt,
   - Erzeugen von MRT-Bildern aus den empfangenen MRT-Aufnahmen, wobei das erste MRT-Bild durch Subtraktion der ersten MRT-Aufnahme von der zweiten MRT-Aufnahme berechnet wird, wobei ein zweites MRT-Bild durch Subtraktion der ersten MRT-Aufnahme von der dritten MRT-Aufnahme berechnet wird, wobei ein drittes MRT-Bild durch Subtraktion der ersten MRT-Aufnahme von der dritten MRT-Aufnahme berechnet wird,
   - Anzeigen und/oder Ausgeben eines oder mehrerer der erzeugten MRT-Bilder und/oder Speichern eines oder mehrerer der erzeugten MRT-Bilder in einem Datenspeicher.
15. Ein Kontrastmittel zur Verwendung gemäß einer der Ausführungsformen 13 oder 14, wobei es sich bei dem Kontrastmittel um einen Stoff oder ein Stoffgemisch mit Gadoxetsäure oder einem Salz der Gadoxetsäure als kontrastverstärkenden Wirkstoff, bevorzugt um das Dinatriumsalz der Gadoxetsäure handelt.
16. Ein Kit umfassend ein Kontrastmittel gemäß einer der Ausführungsformen 13 oder 14 und ein Computerprogrammprodukt gemäß einer der Ausführungsformen 8, 9 oder 10.

Die Erfindung wird nachstehend anhand von Figuren näher erläutert, ohne die Erfindung auf die in den Figuren gezeigten Merkmale oder Merkmalskombinationen beschränken zu wollen.

### Es zeigen:

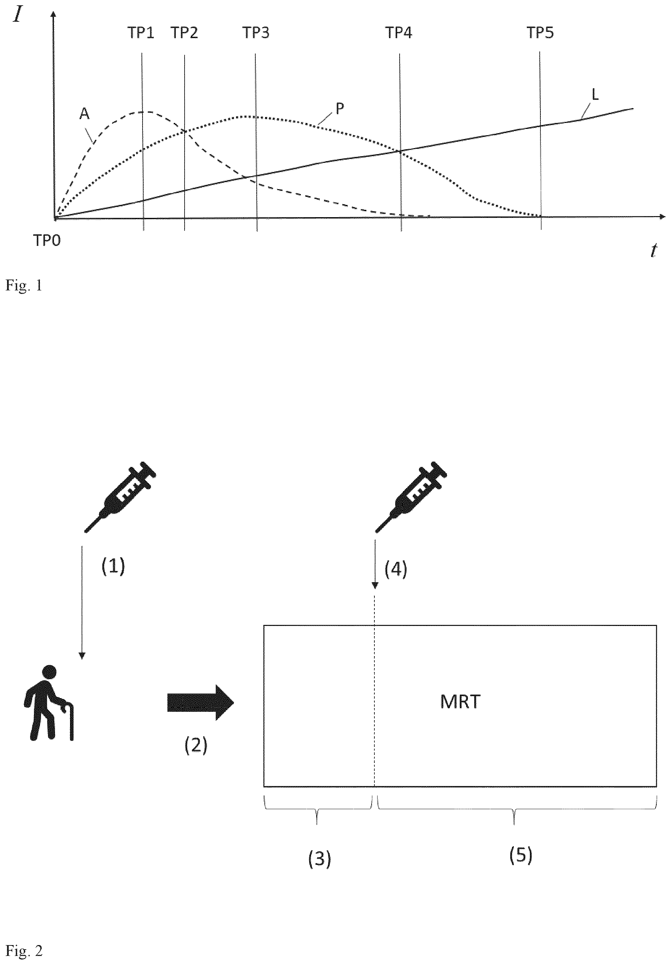

Figur 1 zeigt schematisch den zeitlichen Verlauf der Konzentrationen von Kontrastmittel in den Leberarterien (A), den Lebervenen (P) und den Leberzellen (L). Die Konzentrationen sind in Form der Signalintensitäten I in den genannten Arealen (Leberarterien, Lebervenen, Leberzellen) bei der Magnetresonanzmessung als Funktion der Zeit t dargestellt. Bei einer intravenösen Bolusinjektion steigt die Konzentration des Kontrastmittels in den Leberarterien (A) als erstes an (gestrichelte Kurve). Die Konzentration durchläuft ein Maximum und sinkt dann ab. Die Konzentration in den Lebervenen (P) steigt langsamer an als in den Leberarterien und erreicht ihr Maximum später (gepunktete Kurve). Die Konzentration des Kontrastmittels in den Leberzellen steigt langsam an (durchgezogene Kurve) und erreicht ihr Maximum erst zu einem sehr viel späteren Zeitpunkt (in der Figur 1 nicht dargestellt). Es lassen sich einige charakteristische Zeitpunkte definieren: Zum Zeitpunkt TP0 wird Kontrastmittel intravenös als Bolus appliziert. Zum Zeitpunkt TP1 erreicht die Konzentration (die Signalintensität) des Kontrastmittels in den Leberarterien ihr Maximum. Zum Zeitpunkt TP2 schneiden sich die Kurven der Signalintensitäten bei den Leberarterien und den Lebervenen. Zum Zeitpunkt TP3 durchläuft die Konzentration (die Signalintensität) des Kontrastmittels in den Lebervenen ihr Maximum. Zum Zeitpunkt TP4 schneiden sich die Kurven der Signalintensitäten bei den Leberarterien und den Leberzellen. Zum Zeitpunkt T5 sind die Konzentrationen in den Leberarterien und den Lebervenen auf ein Niveau gesunken, bei dem sie keine messbare Kontrastverstärkung mehr verursachen. Erfindungsgemäß wird Kontrastmittel in Form von zwei Boli verabreicht. Ein erster Bolus mit einem ersten Kontrastmittel wird zum Zeitpunkt TP0 verabreicht, ein zweiter Bolus mit einem zweiten Kontrastmittel zu einem Zeitpunkt nach dem Zeitpunkt TP3, vorzugsweise nach dem Zeitpunkt TP4, noch mehr bevorzugt nach dem Zeitpunkt TP5. Eine erste MRT-Aufnahme wird vorzugsweise nach dem Zeitpunkt TP4, noch mehr bevorzugt nach dem Zeitpunkt TP5 erzeugt. Nach der Applikation des zweiten Kontrastmittels werden zumindest die arterielle Phase und die portalvenöse Phasen ein zweites Mal durchlaufen. Diese Phasen sind durch einen analogen Konzentrationsverlauf von Kontrastmittel in den Arterien und den Lebervenen gekennzeichnet; d.h. die Zeitpunkte TP1 (maximale Kontrastverstärkung der Arterien), TP2 (gleiche Kontrastverstärkung von Arterien und Venen) und TP3 (maximale Kontrastverstärkung der Venen) treten ein zweites Mal auf; sie können als Zeitpunkte TP1', TP2` und TP3' bezeichnet werden. Vorzugsweise werden eine zweite MRT-Aufnahme und ggf. eine dritte MRT-Aufnahme und ggf. weitere MRT-Aufnahmen in einer Zeitspanne zwischen dem Zeitpunkt der Applikation des zweiten Kontrastmittels und dem Zeitpunkt TP3' erzeugt.

Figur 2 zeigt schematisch ein Beispiel einer erfindungsgemäß verkürzten MRT-Untersuchung. Hierbei wird ein erstes Kontrastmittel zunächst in Form eines ersten Bolus appliziert (1). Das Untersuchungsobjekt wird nach einem gewissen Wartezeitraum (2), z.B. 8 bis 20 Minuten, dem MRT zugeführt. Danach wird der MRT-Vorgang gestartet und eine erste MRT-Aufnahme erzeugt (3), die die Leber des Untersuchungsobjekts oder einen Teils davon in der hepatobiliären Phase zeigt. Danach wird dem Untersuchungsobjekt ein zweites Kontrastmittel intravenös in Form einer Bolusinjektion (4) appliziert und anschließend eine oder mehrere weiteres MRT-Aufnahmen der Leber bzw. eines Teils davon in der dynamischen Phase erzeugt.

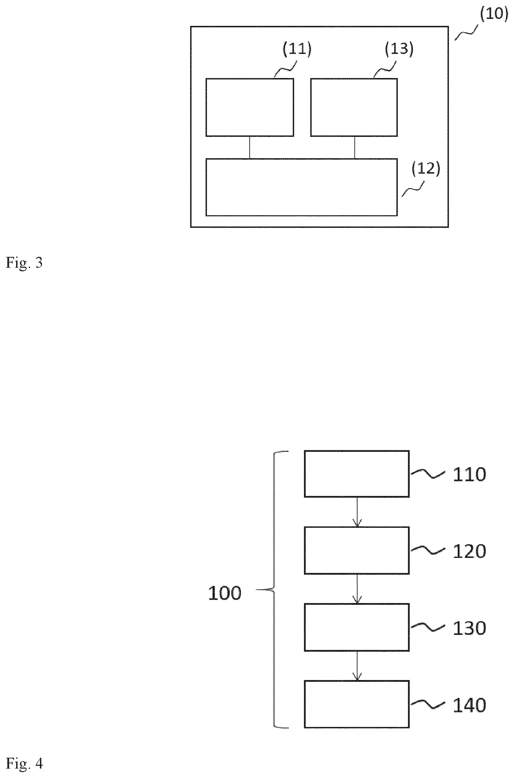

Figur 3 zeigt schematisch eine bevorzugte Ausführungsform des erfindungsgemäßen Systems. Das System (10) umfasst eine Empfangseinheit (11), eine Steuer- und Recheneinheit (12) und eine Ausgabeeinheit (13).

Die Steuer- und Recheneinheit (12) ist konfiguriert, die Empfangseinheit (11) zu veranlassen, eine erste MRT-Aufnahme eines Untersuchungsobjekts zu empfangen, wobei die erste MRT-Aufnahme eine Leber oder einen Teil einer Leber des Untersuchungsobjekts zu einem ersten Zeitpunkt nach einer Applikation eines ersten Kontrastmittels zeigt, wobei gesunde Leberzellen zu dem ersten Zeitpunkt infolge der Applikation des ersten Kontrastmittels in der ersten MRT-Aufnahme kontrastverstärkt dargestellt sind.

Die Steuer- und Recheneinheit (12) ist ferner konfiguriert, die Empfangseinheit (11) zu veranlassen, eine zweite MRT-Aufnahme eines Untersuchungsobjekts zu empfangen, wobei die zweite MRT-Aufnahme dieselbe Leber oder denselben Teil der Leber zu einem zweiten Zeitpunkt nach einer Applikation eines zweiten Kontrastmittels zeigt, wobei gesunde Leberzellen zu dem zweiten Zeitpunkt infolge der Applikation des ersten Kontrastmittels in der zweiten MRT-Aufnahme kontrastverstärkt dargestellt sind, und wobei Blutgefäße zu dem zweiten Zeitpunkt infolge der Applikation des zweiten Kontrastmittels in der zweiten MRT-Aufnahme kontrastverstärkt dargestellt sind.

Die Steuer- und Recheneinheit (12) ist ferner konfiguriert, aus der ersten MRT-Aufnahme und der zweiten MRT-Aufnahme ein erstes MRT-Bild zu erzeugen, wobei das erste MRT-Bild dieselbe Leber oder denselben Teil der Leber zu dem zweiten Zeitpunkt zeigt, wobei der Kontrast zwischen gesunden Leberzellen und Blutgefäßen in dem ersten MRT-Bild gegenüber der zweiten MRT-Aufnahme vergrößert ist,
Die Steuer- und Recheneinheit (12) ist ferner konfiguriert, die Ausgabeeinheit (13) zu veranlassen, das erste MRT-Bild anzuzeigen, auszugeben oder in einem Datenspeicher zu speichern.

Figur 4 zeigt schematisch und beispielhaft eine Ausführungsform des erfindungsgemäßen Verfahrens. Das Verfahren (100) umfasst die Schritte:
(110) Empfangen einer ersten MRT-Aufnahme eines Untersuchungsobjekts, wobei die erste MRT-Aufnahme eine Leber oder einen Teil einer Leber des Untersuchungsobjekts zu einem ersten Zeitpunkt nach einer Applikation eines ersten Kontrastmittels zeigt, wobei gesunde Leberzellen zu dem ersten Zeitpunkt infolge der Applikation des ersten Kontrastmittels in der ersten MRT-Aufnahme kontrastverstärkt dargestellt sind,
(120) Empfangen einer zweiten MRT-Aufnahme desselben Untersuchungsobjekts, wobei die zweite MRT-Aufnahme dieselbe Leber oder denselben Teil der Leber zu einem zweiten Zeitpunkt nach einer Applikation eines zweiten Kontrastmittels zeigt, wobei gesunde Leberzellen zu dem zweiten Zeitpunkt infolge der Applikation des ersten Kontrastmittels in der zweiten MRT-Aufnahme kontrastverstärkt dargestellt sind, und wobei Blutgefäße zu dem zweiten Zeitpunkt infolge der Applikation des zweiten Kontrastmittels in der zweiten MRT-Aufnahme kontrastverstärkt dargestellt sind,
(130) Erzeugen eines ersten MRT-Bildes aus der ersten MRT-Aufnahme und der zweiten MRT-Aufnahme, wobei das erste MRT-Bild dieselbe Leber oder denselben Teil der Leber zu dem zweiten Zeitpunkt zeigt, wobei der Kontrast zwischen gesunden Leberzellen und Blutgefäßen in dem ersten MRT-Bild gegenüber der zweiten MRT-Aufnahme vergrößert ist,
(140) Anzeigen und/oder Ausgeben des ersten MRT-Bildes und/oder Speichern des ersten MRT-Bildes in einem Datenspeicher.

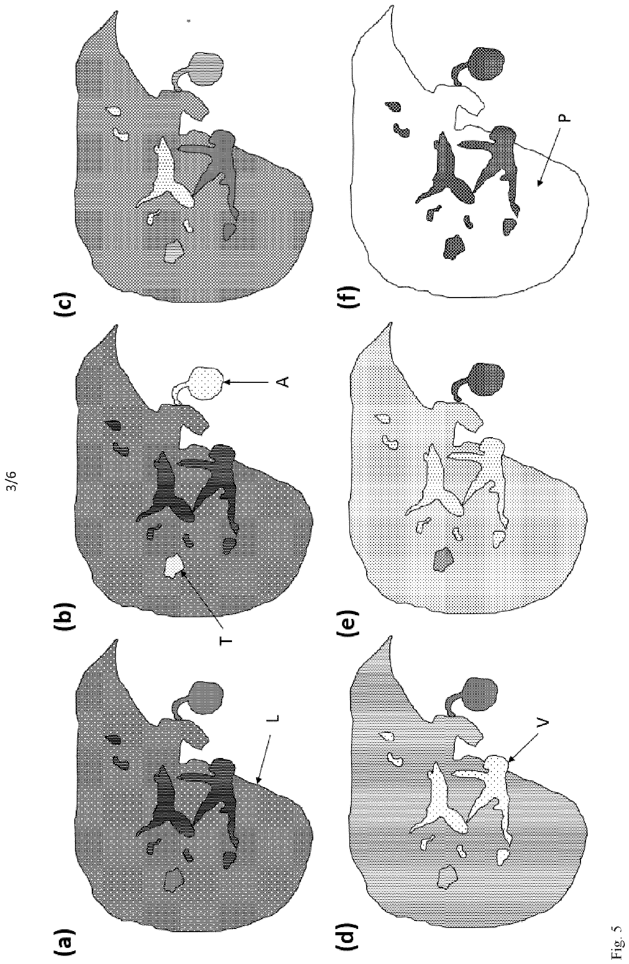

Figur 5 zeigt MRT-Aufnahmen einer Leber während der dynamischen und der hepatobiliären Phase. In den Figuren 5 (a), 5 (b), 5 (c), 5 (d), 5 (e) und 5 (f) ist stets derselbe Querschnitt durch die Leber zu unterschiedlichen Zeitpunkten dargestellt. Die in den Figuren 5 (a), 5 (b), 5 (d) und 5 (f) eingezeichneten Bezugszeichen gelten für alle Figuren 5 (a), 5 (b), 5 (c), 5 (d), 5 (e) und 5 (f); sie sind lediglich der besseren Übersicht halber jeweils nur einmal eingezeichnet.

Fig. 5 (a) zeigt den Querschnitt durch die Leber (L) vor der intravenösen Applikation eines (hepatobiliären) Kontrastmittels. Zu einem Zeitpunkt, der zwischen den Zeitpunkten liegt, die durch die Figuren 5 (a) und 5 (b) dargestellt werden, wurde ein hepatobiliäres Kontrastmittel intravenös als Bolus verabreicht. Dieses erreicht in Fig. 5 (b) über die Leberarterie (A) die Leber. Dementsprechend wird die Leberarterie signalverstärkt dargestellt (arterielle Phase). Ein Tumor (T), der hauptsächlich über Arterien mit Blut versorgt wird, hebt sich ebenfalls als hellerer (signalverstärkter) Bereich vor dem Leberzellengewebe ab. Zu dem Zeitpunkt, der in Figur 5 (c) dargestellt ist, erreicht das hepatobiliäre Kontrastmittel die Leber über die Venen. In Figur 5 (d) heben sich die venösen Blutgefäße (V) als helle (signalverstärkte) Bereiche von dem Lebergewebe ab (portalvenöse Phase). Gleichzeitig steigt die Signalintensität in den gesunden Leberzellen, die hauptsächlich über die Venen mit Kontrastmittel versorgt werden, kontinuierlich an (Fig. 5 (c) → 5 (d) ---> 5 (e) ---> 5 (f)). In der hepatobiliären Phase, die in Fig. 5 (f) dargestellt ist, sind die Leberzellen (P) signalverstärkt dargestellt; die Blutgefäße und der Tumor weisen kein Kontrastmittel mehr auf und sind entsprechend dunkel dargestellt.

Erfindungsgemäß wird zu einem ersten Zeitpunkt während der hepatobiliären Phase eine erste MRT-Aufnahme erzeugt. Dabei kann es sich beispielsweise um die in Fig. 5 (f) gezeigte Aufnahme handeln.

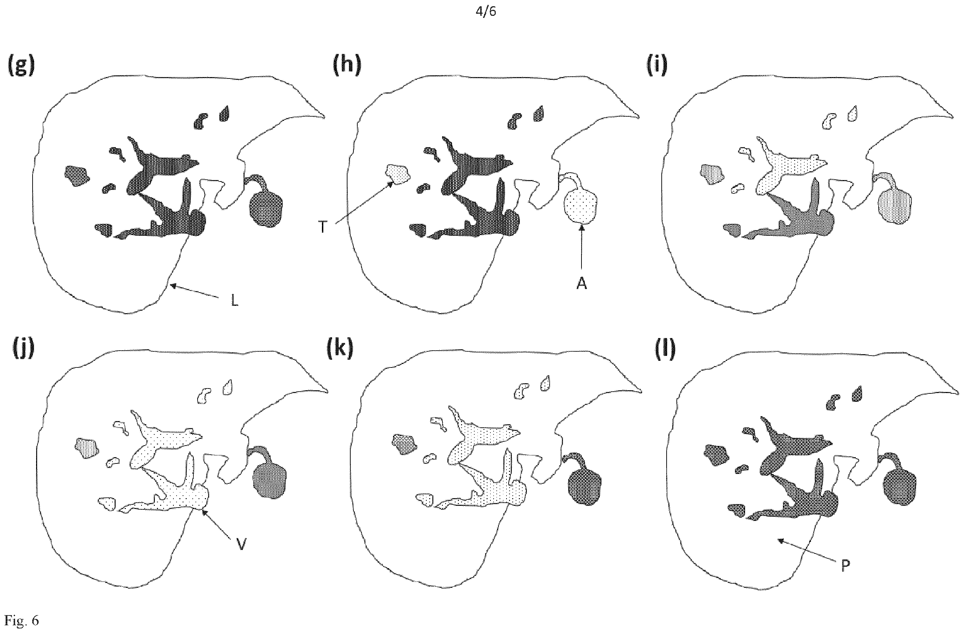

Fig. 6 zeigt dieselbe Leber (L), die bereits in Fig. 5 dargestellt ist. Der in Fig. 6 (g) gezeigte Zustand schließt zeitlich an den in Fig. 5 (f) gezeigten Zustand an. Die Leber ist noch immer infolge der Applikation des hepatobiliären Kontrastmittels signalverstärkt dargestellt. Zu dem in Fig. 6 (g) gezeigten Zeitpunkt wird ein zweites Mal Kontrastmittel in Form eines Bolus intravenös appliziert. Wiederum werden die bereits im Zusammenhang mit Fig. 5 beschriebenen Phasen durchlaufen: die arterielle Phase (Fig. 6 (h)), die portalvenöse Phase (Fig. 6 (j)) und die Spätphase (Fig. 6 (k)). Handelt es sich bei dem zweiten Kontrastmittel ebenfalls um ein hepatobiliäres Kontrastmittel, wird auch eine zweite hepatobiliäre Phase durchlaufen (Fig. 6 (1)), mit dem Unterschied, dass die gesunden Leberzellen noch erstes Kontrastmittel aus der ersten Applikation enthalten und über alle Phasen signalverstärkt dargestellt sind. Dementsprechend ist der Kontrast zwischen Blutgefäßen und den gesunden Leberzellen insbesondere in den in Fig. 6 (i) und Fig 6 (j) gezeigten Aufnahmen geringer als in den in Fig. 5 (c) und Fig. 5 (d) gezeigten Aufnahmen.

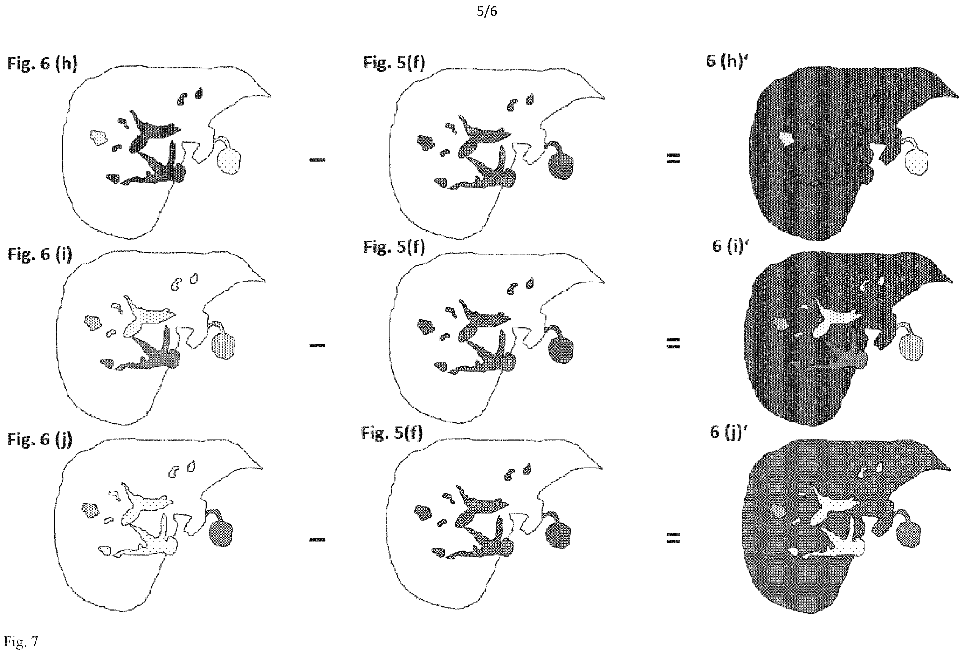

Um diesen Kontrast zu erhöhen, werden für eine oder mehrere der in den Fig. 6 (g), Fig. 6 (h), Fig. 6(i), Fig. 6 (j) und Fig. 6 (k) gezeigten Aufnahmen MRT-Bilder erzeugt. Dies ist beispielhaft in Fig. 7 dargestellt.

Fig. 7 zeigt schematisch und beispielhaft die Erzeugung von drei MRT-Bildern 6 (h)', 6 (i)` und 6 (j)`. Das MRT-Bild 6 (h)` wird durch Subtraktion der MRT-Aufnahme aus Fig. 5 (f) von der MRT-Aufnahme aus Fig. 6 (h) erzeugt. Das MRT-Bild 6 (i)` wird durch Subtraktion der MRT-Aufhahme aus Fig. 5 (f) von der MRT-Aufnahme aus Fig. 6 (i) erzeugt. Das MRT-Bild 6 (j)' wird durch Subtraktion der MRT-Aufnahme aus Fig. 5 (f) von der MRT-Aufnahme aus Fig. 6 (j) erzeugt. Die Subtraktion erfolgt jeweils für die Tonwerte der einzelnen Bildpunkte der MRT-Aufnahmen. MRT-Bild 6 (h)` entspricht der in Fig. 5 (b) gezeigten MRT-Aufnahme; MRT-Bild 6 (1)' entspricht der in Fig. 5 (c) gezeigten MRT-Aufnahme; MRT-Bild 6 (j)` entspricht der in Fig. 5 (d) gezeigten MRT-Aufnahme.

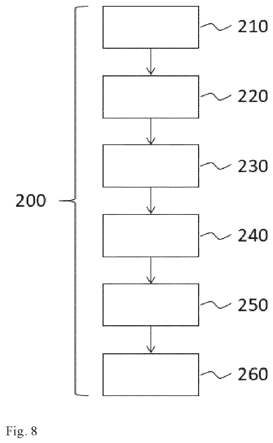

Fig. 8 zeigt beispielhaft und schematisch in Form eines Ablaufdiagramms eine bevorzugte Ausführungsform des erfindungsgemäßen Verfahrens. Das Verfahren (200) umfasst die Schritte:
(210) Empfangen einer ersten MRT-Aufhahme eines Untersuchungsobjekts, wobei die erste MRT-Aufnahme eine Leber oder einen Teil einer Leber des Untersuchungsobjekts zu einem ersten Zeitpunkt nach einer Applikation eines ersten Kontrastmittels während einer ersten hepatobiliären Phase zeigt,
(220) Empfangen einer zweiten MRT-Aufnahme desselben Untersuchungsobjekts, wobei die zweite MRT-Aufnahme dieselbe Leber oder denselben Teil der Leber zu einem zweiten Zeitpunkt nach einer Applikation eines zweiten Kontrastmittels während einer zweiten arteriellen Phase zeigt,
(230) Empfangen einer dritten MRT-Aufnahme desselben Untersuchungsobjekts, wobei die dritte MRT-Aufnahme dieselbe Leber oder denselben Teil der Leber zu einem dritten Zeitpunkt nach der Applikation des zweiten Kontrastmittels während einer zweiten portalvenösen Phase zeigt,
(240) Empfangen einer vierten MRT-Aufnahme desselben Untersuchungsobjekts, wobei die vierte MRT-Aufnahme dieselbe Leber oder denselben Teil der Leber zu einem vierten Zeitpunkt nach der Applikation des zweiten Kontrastmittels während einer zweiten Spätphase zeigt,
(250) Erzeugen von MRT-Bildern aus den empfangenen MRT-Aufnahmen, wobei ein erstes MRT-Bild durch Subtraktion der ersten MRT-Aufnahme von der zweiten MRT-Aufnahme berechnet wird, wobei ein zweites MRT-Bild durch Subtraktion der ersten MRT-Aufnahme von der dritten MRT-Aufhahme berechnet wird, wobei ein drittes MRT-Bild durch Subtraktion der ersten MRT-Aufhahme von der dritten MRT-Aufnahme berechnet wird,
(260) Anzeigen und/oder Ausgeben eines oder mehrerer der erzeugten MRT-Bilder und/oder Speichern eines oder mehrerer der erzeugten MRT-Bilder in einem Datenspeicher.

## Patentansprüche

1. Computer-implementiertes Verfahren umfassend die Schritte
- Empfangen einer ersten MRT-Aufnahme eines Untersuchungsobjekts, wobei die erste MRT-Aufnahme eine Leber oder einen Teil einer Leber des Untersuchungsobjekts zu einem ersten Zeitpunkt nach einer Applikation eines ersten Kontrastmittels zeigt, wobei gesunde Leberzellen zu dem ersten Zeitpunkt infolge der Applikation des ersten Kontrastmittels in der ersten MRT-Aufnahme kontrastverstärkt dargestellt sind,
- Empfangen einer zweiten MRT-Aufnahme desselben Untersuchungsobjekts, wobei die zweite MRT-Aufnahme dieselbe Leber oder denselben Teil der Leber zu einem zweiten Zeitpunkt nach einer Applikation eines zweiten Kontrastmittels zeigt, wobei gesunde Leberzellen zu dem zweiten Zeitpunkt infolge der Applikation des ersten Kontrastmittels in der zweiten MRT-Aufnahme kontrastverstärkt dargestellt sind, und wobei Blutgefäße zu dem zweiten Zeitpunkt infolge der Applikation des zweiten Kontrastmittels in der zweiten MRT-Aufnahme kontrastverstärkt dargestellt sind,
- Erzeugen eines ersten MRT-Bildes aus der ersten MRT-Aufnahme und der zweiten MRT-Aufnahme, wobei das erste MRT-Bild dieselbe Leber oder denselben Teil der Leber zu dem zweiten Zeitpunkt zeigt, wobei der Kontrast zwischen gesunden Leberzellen und Blutgefäßen in dem ersten MRT-Bild gegenüber der zweiten MRT-Aufnahme vergrößert ist,
- Anzeigen und/oder Ausgeben des ersten MRT-Bildes und/oder Speichern des ersten MRT-Bildes in einem Datenspeicher.

2. Verfahren gemäß Anspruch 1, umfassend die Schritte
- Empfangen der ersten MRT-Aufnahme des Untersuchungsobjekts, wobei die erste MRT-Aufnahme die Leber oder den Teil der Leber des Untersuchungsobjekts zu dem ersten Zeitpunkt nach der Applikation des ersten Kontrastmittels zeigt, wobei gesunde Leberzellen zu dem ersten Zeitpunkt infolge der Applikation des ersten Kontrastmittels in der ersten MRT-Aufnahme kontrastverstärkt dargestellt sind,
- Empfangen der zweiten MRT-Aufnahme desselben Untersuchungsobjekts, wobei die zweite MRT-Aufnahme dieselbe Leber oder denselben Teil der Leber zu dem zweiten Zeitpunkt nach der Applikation des zweiten Kontrastmittels zeigt, wobei gesunde Leberzellen zu dem zweiten Zeitpunkt infolge der Applikation des ersten Kontrastmittels in der zweiten MRT-Aufnahme kontrastverstärkt dargestellt sind, und wobei Blutgefäße zu dem zweiten Zeitpunkt infolge der Applikation des zweiten Kontrastmittels in der zweiten MRT-Aufhahme kontrastverstärkt dargestellt sind,
- Erzeugen des ersten MRT-Bildes aus der ersten MRT-Aufnahme und der zweiten MRT-Aufnahme, wobei das erste MRT-Bild dieselbe Leber oder denselben Teil der Leber nach der Applikation des zweiten Kontrastmittels zeigt, wobei die durch das erste Kontrastmittel verursachte Kontrastverstärkung gesunder Leberzellen eliminiert ist,
- Anzeigen und/oder Ausgeben des ersten MRT-Bildes und/oder Speichern des ersten MRT-Bildes in einem Datenspeicher.

3. Verfahren gemäß einem der Ansprüche 1 oder 2, umfassend die Schritte
- Empfangen der ersten MRT-Aufnahme des Untersuchungsobjekts, wobei die erste MRT-Aufnahme die Leber oder den Teil einer Leber des Untersuchungsobjekts zu dem ersten Zeitpunkt nach der Applikation des ersten Kontrastmittels während einer ersten hepatobiliären Phase zeigt,
- Empfangen der zweiten MRT-Aufnahme desselben Untersuchungsobjekts, wobei die zweite MRT-Aufnahme dieselbe Leber oder denselben Teil der Leber zu dem zweiten Zeitpunkt nach der Applikation des zweiten Kontrastmittels während einer zweiten arteriellen Phase zeigt,
- optionales Empfangen einer dritten MRT-Aufnahme desselben Untersuchungsobjekts, wobei die dritte MRT-Aufnahme dieselbe Leber oder denselben Teil der Leber zu einem dritten Zeitpunkt nach der Applikation des zweiten Kontrastmittels während einer zweiten portalvenösen Phase zeigt,
- optionales Empfangen einer vierten MRT-Aufnahme desselben Untersuchungsobjekts, wobei die vierte MRT-Aufnahme dieselbe Leber oder denselben Teil der Leber zu einem vierten Zeitpunkt nach der Applikation des zweiten Kontrastmittels während einer zweiten Spätphase zeigt,
- Erzeugen von MRT-Bildern aus den empfangenen MRT-Aufnahmen, wobei das erste MRT-Bild durch Subtraktion der ersten MRT-Aufnahme von der zweiten MRT-Aufnahme berechnet wird, wobei optional ein zweites MRT-Bild durch Subtraktion der ersten MRT-Aufnahme von der dritten MRT-Aufnahme berechnet wird, wobei optional ein drittes MRT-Bild durch Subtraktion der ersten MRT-Aufnahme von der dritten MRT-Aufhahme berechnet wird,
- Anzeigen und/oder Ausgeben eines oder mehrerer der erzeugten MRT-Bilder und/oder Speichern eines oder mehrerer der erzeugten MRT-Bilder in einem Datenspeicher.

4. Verfahren gemäß einem der Ansprüche 1 oder 2, umfassend die Schritte
- Empfangen der ersten MRT-Aufnahme des Untersuchungsobjekts, wobei die erste MRT-Aufnahme die Leber oder den Teil der Leber des Untersuchungsobjekts zu dem ersten Zeitpunkt nach der Applikation des ersten Kontrastmittels zeigt, wobei gesunde Leberzellen zu dem ersten Zeitpunkt infolge der Applikation des ersten Kontrastmittels in der ersten MRT-Aufnahme kontrastverstärkt dargestellt sind,
- Empfangen der zweiten MRT-Aufnahme desselben Untersuchungsobjekts, wobei die zweite MRT-Aufnahme dieselbe Leber oder denselben Teil der Leber zu dem zweiten Zeitpunkt nach der Applikation des zweiten Kontrastmittels zeigt, wobei gesunde Leberzellen zu dem zweiten Zeitpunkt infolge der Applikation des ersten Kontrastmittels in der zweiten MRT-Aufnahme kontrastverstärkt dargestellt sind, und wobei Blutgefäße zu dem zweiten Zeitpunkt infolge der Applikation des zweiten Kontrastmittels in der zweiten MRT-Aufnahme kontrastverstärkt dargestellt sind,
- Zuführen der ersten MRT-Aufnahme und der zweiten MRT-Aufnahme einem künstlichen neuronalen Netzes, wobei das künstliche neuronal Netz in einem überwachten Lemverfahren trainiert worden ist, aus ersten Referenz-MRT-Aufnahmen und zweiten Referenz-MRT-Aufnahmen dritte Referenz-MRT-Aufnahmen zu erzeugen, wobei jede erste Referenz-MRT-Aufnahme eine Leber oder einen Teil einer Leber des Untersuchungsobjekts zu einem ersten Zeitpunkt nach einer Applikation eines ersten Kontrastmittels zeigt, wobei gesunde Leberzellen zu dem ersten Zeitpunkt infolge der Applikation des ersten Kontrastmittels in der ersten Referenz-MRT-Aufnahme kontrastverstärkt dargestellt sind, wobei jede zweite Referenz-MRT-Aufnahme dieselbe Leber oder denselben Teil der Leber zu einem zweiten Zeitpunkt nach einer Applikation eines zweiten Kontrastmittels zeigt, wobei gesunde Leberzellen zu dem zweiten Zeitpunkt infolge der Applikation des ersten Kontrastmittels in der zweiten MRT-Aufnahmε kontrastverstärkt dargestellt sind, und wobei Blutgefäße zu dem zweiten Zeitpunkt infolge der Applikation des zweiten Kontrastmittels in der zweiten MRT-Aufhahme kontrastverstärkt dargestellt sind, und wobei jede dritte Referenz-MRT-Aufnahme dieselbe Leber oder denselben Teil der Leber zu dem zweiten Zeitpunkt nach der Applikation des zweiten Kontrastmittels zeigt, wobei nur Blutgefäße zu dem zweiten Zeitpunkt infolge der Applikation des zweiten Kontrastmittels in der zweiten MRT-Aufnahme kontrastverstärkt dargestellt sind, wobei gesunde Leberzellen nicht kontrastverstärkt dargestellt sind,
- Anzeigen und/oder Ausgeben des ersten MRT-Bildes und/oder Speichern des ersten MRT-Bildes in einem Datenspeicher.

5. Verfahren gemäß einem der Ansprüche 1 oder 2, wobei es sich bei dem ersten Kontrastmittel um ein hepatobiliäres Kontrastmittel, vorzugsweise um einen Stoff oder ein Stoffgemisch mit Gadoxetsäure oder einem Salz der Gadoxetsäure als kontrastverstärkenden Wirkstoff handelt, und es sich bei dem zweiten Kontrastmittel entweder um das gleiche Kontrastmittel wie das erste Kontrastmittel handelt oder es sich bei dem zweiten Kontrastmittel um ein extrazelluläres Kontrastmittel, vorzugsweise um einen Stoff oder ein Stoffgemisch mit Gadobutrol, Gadoteridol, Gadotersäure, Gadopentetsäure oder Gadodiamid als kontrastverstärkenden Wirkstoff handelt.

6. System umfassend
• eine Empfangseinheit,
• eine Steuer- und Recheneinheit und
• eine Ausgabeeinheit,
- wobei die Steuer- und Recheneinheit konfiguriert ist, die Empfangseinheit zu veranlassen, eine erste MRT-Aufnahme eines Untersuchungsobjekts zu empfangen, wobei die erste MRT-Aufnahme eine Leber oder einen Teil einer Leber des Untersuchungsobjekts zu einem ersten Zeitpunkt nach einer Applikation eines ersten Kontrastmittels zeigt, wobei gesunde Leberzellen zu dem ersten Zeitpunkt infolge der Applikation des ersten Kontrastmittels in der ersten MRT-Aufnahme kontrastverstärkt dargestellt sind,
- wobei die Steuer- und Recheneinheit konfiguriert ist, die Empfangseinheit zu veranlassen, eine zweite MRT-Aufnahme eines Untersuchungsobjekts zu empfangen, wobei die zweite MRT-Aufnahme dieselbe Leber oder denselben Teil der Leber zu einem zweiten Zeitpunkt nach einer Applikation eines zweiten Kontrastmittels zeigt, wobei gesunde Leberzellen zu dem zweiten Zeitpunkt infolge der Applikation des ersten Kontrastmittels in der zweiten MRT-Aufnahme kontrastverstärkt dargestellt sind, und wobei Blutgefäße zu dem zweiten Zeitpunkt infolge der Applikation des zweiten Kontrastmittels in der zweiten MRT-Aufnahme kontrastverstärkt dargestellt sind,
- wobei die Steuer- und Recheneinheit konfiguriert ist, aus der ersten MRT-Aufnahme und der zweiten MRT-Aufnahme ein erstes MRT-Bild zu erzeugen, wobei das erste MRT-Bild dieselbe Leber oder denselben Teil der Leber zu dem zweiten Zeitpunkt zeigt, wobei der Kontrast zwischen gesunden Leberzellen und Blutgefäßen in dem ersten MRT-Bild gegenüber der zweiten MRT-Aufnahme vergrößert ist,
- wobei die Steuer- und Recheneinheit konfiguriert ist, die Ausgabeeinheit zu veranlassen, das erste MRT-Bild anzuzeigen, auszugeben oder in einem Datenspeicher zu speichern.

7. Computersystem gemäß Anspruch 6,
- wobei die Steuer- und Recheneinheit konfiguriert ist, die Empfangseinheit zu veranlassen, eine erste MRT-Aufnahme, eine zweite MRT-Aufhahme, eine dritte MRT-Aufnahme und eine vierte MRT-Aufhahme zu empfangen,
- wobei die erste MRT-Aufnahme die Leber oder den Teil der Leber des Untersuchungsobjekts zu dem ersten Zeitpunkt nach der Applikation des ersten Kontrastmittels während einer ersten hepatobiliären Phase zeigt,
- wobei die zweite MRT-Aufnahme dieselbe Leber oder denselben Teil der Leber zu einem zweiten Zeitpunkt nach der Applikation des zweiten Kontrastmittels während einer zweiten arteriellen Phase zeigt,
- wobei die dritte MRT-Aufnahme dieselbe Leber oder denselben Teil der Leber zu einem dritten Zeitpunkt nach der Applikation des zweiten Kontrastmittels während einer zweiten portalvenösen Phase zeigt,
- wobei die vierte MRT-Aufnahme dieselbe Leber oder denselben Teil der Leber zu einem vierten Zeitpunkt nach der Applikation des zweiten Kontrastmittels während einer zweiten Spätphase zeigt,
- wobei die Steuer- und Recheneinheit konfiguriert ist, MRT-Bilder aus den empfangenen MRT-Aufnahmen zu berechnen, wobei ein erstes MRT-Bild durch Subtraktion der ersten MRT-Aufnahme von der zweiten MRT-Aufnahme berechnet wird, wobei ein zweites MRT-Bild durch Subtraktion der ersten MRT-Aufnahme von der dritten MRT-Aufnahme berechnet wird, wobei ein drittes MRT-Bild durch Subtraktion der ersten MRT-Aufnahme von der dritten MRT-Aufnahme berechnet wird,
- wobei die Steuer- und Recheneinheit konfiguriert ist, die Ausgabeeinheit zu veranlassen, ein oder mehrere der berechneten MRT-Bilder auf einem Bildschirm anzuzeigen und/oder auf einem Drucker auszugeben und/oder in einem Datenspeicher zu speichern.

8. Computerprogrammprodukt umfassend ein Computerprogramm, das in einen Arbeitsspeicher eines Computersystems geladen werden kann und dort das Computersystem dazu veranlasst, folgende Schritte ausführen:
- Empfangen einer ersten MRT-Aufhahme eines Untersuchungsobjekts, wobei die erste MRT-Aufnahme eine Leber oder einen Teil einer Leber des Untersuchungsobjekts zu einem ersten Zeitpunkt nach einer Applikation eines ersten Kontrastmittels zeigt, wobei gesunde Leberzellen zu dem ersten Zeitpunkt infolge der Applikation des ersten Kontrastmittels in der ersten MRT-Aufnahme kontrastverstärkt dargestellt sind,
- Empfangen einer zweiten MRT-Aufnahme, wobei die zweite MRT-Aufnahme dieselbe Leber oder denselben Teil der Leber zu einem zweiten Zeitpunkt nach einer Applikation eines zweiten Kontrastmittels zeigt, wobei gesunde Leberzellen zu dem zweiten Zeitpunkt infolge der Applikation des ersten Kontrastmittels in der zweiten MRT-Aufnahme kontrastverstärkt dargestellt sind, und wobei Blutgefäße zu dem zweiten Zeitpunkt infolge der Applikation des zweiten Kontrastmittels in der zweiten MRT-Aufnahme kontrastverstärkt dargestellt sind,
- Erzeugen eines ersten MRT-Bildes aus der ersten MRT-Aufhahme und der zweiten MRT-Aufnahme, wobei das erste MRT-Bild dieselbe Leber oder denselben Teil der Leber zu dem zweiten Zeitpunkt zeigt, wobei der Kontrast zwischen gesunden Leberzellen und Blutgefäßen in dem ersten MRT-Bild gegenüber der zweiten MRT-Aufnahme vergrößert ist,
- Anzeigen und/oder Ausgeben des ersten MRT-Bildes und/oder Speichern des ersten MRT-Bildes in einem Datenspeicher.

9. Computerprogrammprodukt gemäß Anspruch 8, wobei das Computerprogramm das Computersystem dazu veranlasst die folgenden Schritte auszuführen:
- Empfangen der ersten MRT-Aufnahme des Untersuchungsobjekts, wobei die erste MRT-Aufnahme die Leber oder den Teil der Leber des Untersuchungsobjekts zu dem ersten Zeitpunkt nach der Applikation des ersten Kontrastmittels während einer ersten hepatobiliären Phase zeigt,
- Empfangen der zweiten MRT-Aufhahme, wobei die zweite MRT-Aufnahme dieselbe Leber oder denselben Teil der Leber zu dem zweiten Zeitpunkt nach der Applikation des Kontrastmittels zweiten während einer zweiten arteriellen Phase zeigt,
- Empfangen einer dritten MRT-Aufnahme, wobei die dritte MRT-Aufnahme dieselbe Leber oder denselben Teil der Leber zu einem dritten Zeitpunkt nach der Applikation des zweiten Kontrastmittels während einer zweiten portalvenösen Phase zeigt,
- Empfangen einer vierten MRT-Aufnahme, wobei die vierte MRT-Aufnahme dieselbe Leber oder denselben Teil der Leber zu einem vierten Zeitpunkt nach der Applikation des zweiten Kontrastmittels während einer zweiten Spätphase zeigt,
- Erzeugen von MRT-Bildern aus den empfangenen MRT-Aufnahmen, wobei das erste MRT-Bild durch Subtraktion der ersten MRT-Aufhahme von der zweiten MRT-Aufnahme berechnet wird, wobei ein zweites MRT-Bild durch Subtraktion der ersten MRT-Aufnahme von der dritten MRT-Aufnahme berechnet wird, wobei ein drittes MRT-Bild durch Subtraktion der ersten MRT-Aufnahme von der dritten MRT-Aufnahme berechnet wird,
- Anzeigen und/oder Ausgeben eines oder mehrerer der erzeugten MRT-Bilder und/oder Speichern eines oder mehrerer der erzeugten MRT-Bilder in einem Datenspeicher.

10. Computerprogrammprodukt gemäß Anspruch 9, wobei das Computerprogramm das Computersystem dazu veranlasst, einen oder mehrere der in den Ansprüchen 1 bis 5 aufgeführten Schritte auszuführen.

11. Verwendung eines ersten und eines zweiten Kontrastmittels in einem MRT-Verfahren, wobei das MRT-Verfahren die folgenden Schritte umfasst:
- Applizieren des ersten Kontrastmittels,
- Erzeugen einer ersten MRT-Aufnahme, wobei die erste MRT-Aufnahme eine Leber oder einen Teil einer Leber zu einem ersten Zeitpunkt nach der Applikation des ersten Kontrastmittels zeigt, wobei gesunde Leberzellen zu dem ersten Zeitpunkt infolge der Applikation des ersten Kontrastmittels in der ersten MRT-Aufnahme kontrastverstärkt dargestellt sind,
- Applizieren des zweiten Kontrastmittels zu einem Zeitpunkt nach dem Erzeugen der ersten MRT-Aufnahme,
- Erzeugen einer zweiten MRT-Aufnahme, wobei die zweite MRT-Aufhahme dieselbe Leber oder denselben Teil der Leber zu einem zweiten Zeitpunkt nach der Applikation des zweiten Kontrastmittels zeigt, wobei gesunde Leberzellen zu dem zweiten Zeitpunkt infolge der Applikation des ersten Kontrastmittels in der zweiten MRT-Aufnahme kontrastverstärkt dargestellt sind, und wobei Blutgefäße zu dem zweiten Zeitpunkt infolge der Applikation des zweiten Kontrastmittels in der zweiten MRT-Aufhahme kontrastverstärkt dargestellt sind,
- Erzeugen eines ersten MRT-Bildes aus der ersten MRT-Aufnahme und der zweiten MRT-Aufnahme, wobei das erste MRT-Bild dieselbe Leber oder denselben Teil der Leber zu dem zweiten Zeitpunkt zeigt, wobei der Kontrast zwischen gesunden Leberzellen und Blutgefäßen in dem ersten MRT-Bild gegenüber der zweiten MRT-Aufnahme vergrößert ist,
- Anzeigen und/oder Ausgeben eines oder mehrere der erzeugten MRT-Bilder und/oder Speichern eines oder mehrerer der erzeugten MRT-Bilder in einem Datenspeicher.

12. Verwendung gemäß Anspruch 11, umfassend die Schritte:
- Applizieren des ersten Kontrastmittels,
- Erzeugen der ersten MRT-Aufnahme, wobei die erste MRT-Aufnahme die Leber oder den Teil der Leber des Untersuchungsobjekts zu dem ersten Zeitpunkt nach der Applikation des ersten Kontrastmittels während einer ersten hepatobiliären Phase zeigt,
- Applizieren des zweiten Kontrastmittels zu einem Zeitpunkt nach dem Erzeugen der ersten MRT-Aufhahme,
- Erzeugen der zweiten MRT-Aufhahme, wobei die zweite MRT-Aufnahme dieselbe Leber oder denselben Teil der Leber zu dem zweiten Zeitpunkt nach der Applikation des zweiten Kontrastmittels während einer zweiten arteriellen Phase zeigt,
- Erzeugen einer dritten MRT-Aufnahme, wobei die dritte MRT-Aufnahme dieselbe Leber oder denselben Teil der Leber zu einem dritten Zeitpunkt nach der Applikation des zweiten Kontrastmittels während einer zweiten portalvenösen Phase zeigt,
- Erzeugen einer vierten MRT-Aufhahme, wobei die vierte MRT-Aufhahme dieselbe Leber oder denselben Teil der Leber zu einem vierten Zeitpunkt nach der Applikation des zweiten Kontrastmittels während einer zweiten Spätphase zeigt,
- Erzeugen von MRT-Bildern aus den empfangenen MRT-Aufnahmen, wobei das erste MRT-Bild durch Subtraktion der ersten MRT-Aufnahme von der zweiten MRT-Aufnahme berechnet wird, wobei ein zweites MRT-Bild durch Subtraktion der ersten MRT-Aufhahme von der dritten MRT-Aufhahme berechnet wird, wobei ein drittes MRT-Bild durch Subtraktion der ersten MRT-Aufnahme von der dritten MRT-Aufnahme berechnet wird,
- Anzeigen und/oder Ausgeben eines oder mehrerer der erzeugten MRT-Bilder und/oder Speichern eines oder mehrerer der erzeugten MRT-Bilder in einem Datenspeicher.

13. Ein erstes und ein zweites Kontrastmittel zur Verwendung in einem MRT-Verfahren, wobei das MRT-Verfahren die folgenden Schritte umfasst:
- Applizieren des ersten Kontrastmittels,
- Erzeugen einer ersten MRT-Aufnahme, wobei die erste MRT-Aufnahme eine Leber oder einen Teil einer Leber zu einem ersten Zeitpunkt nach der Applikation des ersten Kontrastmittels zeigt, wobei gesunde Leberzellen zu dem ersten Zeitpunkt infolge der Applikation des ersten Kontrastmittels in der ersten MRT-Aufnahme kontrastverstärkt dargestellt sind,
- Applizieren des zweiten Kontrastmittels zu einem Zeitpunkt nach dem Erzeugen der ersten MRT-Aufnahme,
- Erzeugen einer zweiten MRT-Aufnahme, wobei die zweite MRT-Aufnahme dieselbe Leber oder denselben Teil der Leber zu einem zweiten Zeitpunkt nach der Applikation des zweiten Kontrastmittels zeigt, wobei gesunde Leberzellen zu dem zweiten Zeitpunkt infolge der Applikation des ersten Kontrastmittels in der zweiten MRT-Aufnahme kontrastverstärkt dargestellt sind, und wobei Blutgefäße zu dem zweiten Zeitpunkt infolge der Applikation des zweiten Kontrastmittels in der zweiten MRT-Aufnahme kontrastverstärkt dargestellt sind,
- Erzeugen eines ersten MRT-Bildes aus der ersten MRT-Aufnahme und der zweiten MRT-Aufnahme, wobei das erste MRT-Bild dieselbe Leber oder denselben Teil der Leber zu dem zweiten Zeitpunkt zeigt, wobei der Kontrast zwischen gesunden Leberzellen und Blutgefäßen in dem ersten MRT-Bild gegenüber der zweiten MRT-Aufnahme vergrößert ist,
- Anzeigen und/oder Ausgeben eines oder mehrere der erzeugten MRT-Bilder und/oder Speichern eines oder mehrerer der erzeugten MRT-Bilder in einem Datenspeicher.

14. Kontrastmittel zur Verwendung gemäß Anspruch 13, wobei es sich bei dem ersten Kontrastmittel um ein hepatobiliäres Kontrastmittel, vorzugsweise um einen Stoff oder ein Stoffgemisch mit Gadoxetsäure oder einem Salz der Gadoxetsäure als kontrastverstärkenden Wirkstoff handelt, und es sich bei dem zweiten Kontrastmittel entweder um dasselbe Kontrastmittel wie das erste Kontrastmittel handelt oder es sich bei dem zweiten Kontrastmittel um ein extrazelluläres Kontrastmittel, vorzugsweise um einen Stoff oder ein Stoffgemisch mit Gadobutrol, Gadoteridol, Gadotersäure, Gadopentetsäure oder Gadodiamid als kontrastverstärkenden Wirkstoff handelt.

15. Kit umfassend ein Kontrastmittel gemäß Anspruch 13 und ein Computerprogrammprodukt gemäß einem der Ansprüche 8, 9 oder 10.
